# EUROPÄISCHE PATENTSCHRIFT

(11) **EP 0 679 657 B1**
(45) Veröffentlichungstag und Bekanntmachung des Hinweises auf die Patenterteilung: **09.07.2003**
(21) Anmeldenummer: 95810259.2
(22) Anmeldetag: 19.04.1995
(51) Int. Cl.: C07H 19/067, C07H 19/167, C07H 21/00, C12Q 1/68, A61K 31/70

(54) **Nukleoside und Oligonukleotide mit 2'-Ethergruppen**
Nucleosides and oligonucleotides containing 2'-ether groups
Nucléosides et oligonucléotides contenant des groupes 2'-éther

(30) Priorität: 27.04.1994 CH 130794
(43) Veröffentlichungstag der Anmeldung: 02.11.1995
(73) Patentinhaber: Novartis AG, 4056 Basel (CH); Novartis Pharma GmbH, 1230 Wien (AT)
(72) Erfinder: Martin, Pierre, Dr., CH-4310 Rheinfelden (CH)

(56) Entgegenhaltungen:
- EP-A- 0 626 387
- WO-A-91/06556
- TETRAHEDRON LETTERS, Bd. 34, Nr. 6, 1993 OXFORD GB, Seiten 1003-1006, B.C.FROEHLER ET AL. 'Oligonucleotides Derived from 5-(1-Propynyl)-2'-O-allyl-Uridine and 5-(1-Propynyl)-2'-O-Allyl-Cytidine : Synthesis and RNA Duplex Formation.'
- BIOCHEMISTRY, Bd. 32, Nr. 30, 3.August 1993 EASTON, PA US, Seiten 7832-7838, E.A.LESNIK ET AL. 'Oligodeoxynucleotides Containing 2'-O-Modified Adenosine: Synthesis and Effects on Stability of DNA:RNA Duplexes.'
- CHEMICAL ABSTRACTS, vol. 122, no. 5, 30.Januar 1995 Columbus, Ohio, US; abstract no. 56416v, H.KATO ET AL. 'Preparation of O-haloalkyl Nucleosides and Nucleoside Derivatives Having Amino or Thiol Linkers.' Seite 1233; Spalte 2; & JP-A-06 256 380 (TOA GOSEI CHEM. IND.) 13.September 1994

## Beschreibung

Die Erfindung betrifft Ribo-Nukleosidanaloge, deren 2'-OH Gruppe mit Hydroxyethyl- oder Fluoralkylgruppen verethert ist, ein Verfahren zu deren Herstellung, Oligonukleotide mit diesen Nukleosiden und die Verwendung der Nukleoside zur Herstellung von Oligonukleotiden mit gleichen oder verschiedenen Nukleosideinheiten im Molekül.

Nukleoside und Oligonukleotide haben als antivirale Wirkstoffe bzw. wegen ihrer Fähigkeit zur Wechselwirkung mit Nukleinsäuren ("Antisense"-Oligonukleotide) und der damit verbundenen biologischen Aktivität breites Interesse gefunden, siehe zum Beispiel Uhlmann, E., Peyman, A., Chemical Reviews 90:543-584 (1990). Zur Bereitstellung von Nukleosiden mit neuen Eigenschaften oder zur Verbesserung der Wechselwirkung von Antisense-Oligonukleotiden mit natürlichen Nukleinsäuren sowie ihrer Stabilität gegenüber Nukleasen sind die Zuckerreste von Nukleosiden (bzw. der Nukleotideinheiten in Oligonukleotiden), oder die internukleotidische Phosphatbindung in Oligonukleotiden in unterschiedlichster Weise modifiziert worden, siehe zum Beispiel Marquez, V.E., Lim, M.I., Medicinal Research Reviews 6:1-40 (1986), Hélène, C., Toulmé, J.J., Biochimica et Biophysica Acta 1049:99-125 (1990), Englisch, U., Gauss, D.H., Angewandte Chemie 103:629-646 (1991), Matteucci, M.D., Bischofberger, N., Annual Reports in Medicinal Chemistry 26:87-296 (1991). In Cook, P.D., Anti-Cancer Drug Design 6:585-607 (1991) und WO 91/06556 werden Nukleoside beschrieben, die an der 2'-OH-Gruppe des Zuckers modifiziert sind. Die beschriebenen Modifikationen führen zu erhöhter Nukleaseresistenz; je länger der Alkylrest wird, umso höher wird die Nukleaseresistenz. Dabei wird mit kleinen Alkylresten wie Methyl, Ethyl oder Propyl eine schwache Erhöhung der Bindungsaffinität beobachtet, bei längeren Ketten sinkt die Bindungsaffinität aber drastisch ab. In Lesnik, E.A., et al., Biochemistry 32, 7832-7838 (1993) werden Oligonukleotide, mit Nukleosidbausteinen, die an der 2' Position substituiert sind, beschrieben, insbesondere: 2'-F-, 2'-O-Methyl-, 2'-O-Ethyl-, 2'-O-Propyl-, 2'-O-Butyl-, 2'-O-Pentyl-, 2'-O-Nonyl-, 2'-O-Allyl- und 2-O-Benzyl-Substituenten. Dabei wird gezeigt, dass die höchste Duplexstabilität mit einem 2'-F-Substituenten erzielt wird. Nukleoside mit Hydroxyethyl- oder Fluoralkylgruppen als Seitenketten an der 2'-OH Gruppe wurden bis danhin nie in Oligonukleotide eingebaut. Überraschenderweise erhöhen die erfindungsgemässen Modifikationen die Bindungsaffinität zur komplementären RNA gegenüber gleich langen unsubstituierten Alkylketten. Dieses Ergebnis war aufgrund der publizierten Daten nicht zu erwarten. In Analogie zu den 2'-OH-modifizierten Oligoribonukleotiden zeichnen sich die erfindungsgemässen Verbindungen ebenfalls durch eine markante Nukleaseresistenz aus. Zusätzlich weisen Oligonukleotide, welche die erfindungsgemässen Nukleoside enthalten, eine erhöhte zelluläre Aufnahme auf und verfügen demzufolge über eine verbesserte Bio-Verfügbarkeit und Aktivität in vivo.

Ein Gegenstand der Erfindung sind Verbindungen der Formel I 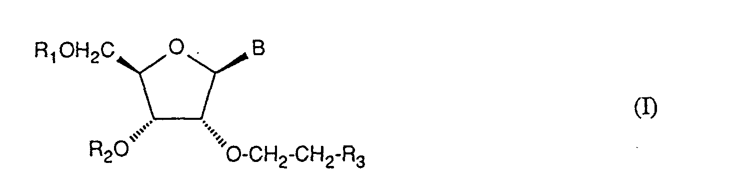 worin R₁ und R₂ unabhängig voneinander für Wasserstoff oder eine Schutzgruppe stehen oder R₁ diese Bedeutungen hat und R₂ für einen eine phosphorhaltige Nukleotid-Brückengruppe bildenden Rest steht; B einen Purin- oder Pyrimidinrest oder ein Analoges davon bedeutet; und R₃ für OH, F oder (CF₂)ₙCF₃ steht, worin n eine Zahl von 0 bis 7 bedeutet.

Wenn R₃ OH bedeutet, kann diese Hydroxygruppe mit einer Gruppe wie für R₁ und R₂ definiert geschützt sein.

In einer bevorzugten Ausführungsform steht n für die Zahl 0.

In einer bevorzugten Ausführungsform stellen R₁ und R₂ Wasserstoff dar.

Schutzgruppen und Verfahren zur Derivatisierung der Hydroxylgruppen mit solchen Schutzgruppen sind in der Zucker- und Nukleotidchemie allgemein bekannt und zum Beispiel von Greene, B.T., Protective Groups in Organic Synthesis, Wiley Interscience, New York (1991), von Sonveaux, E., Bioorganic Chemistry 14:274-325 (1986) oder von Beaucage, S.L., Iyer, R., Tetrahedron 48:2223-2311 (1992) beschrieben. Beispiele für solche Schutzgruppen sind: Benzyl, Methylbenzyl, Dimethylbenzyl, Methoxybenzyl, Dimethoxybenzyl, Brombenzyl, 2,4-Dichlorbenzyl; Diphenylmethyl, Di(methylphenyl)-methyl, Di(dimethylphenyl)methyl, Di(methoxyphenyl)methyl, Di(dimethoxyphenyl)-methyl, Triphenylmethyl, Tris-4,4',4"-tert.butylphenylmethyl, Di-p-anisylphenylmethyl, Tri(methylphenyl)methyl, Tri(dimethylphenyl)methyl, Methoxyphenyl(diphenyl)methyl, Di(methoxyphenyl)phenylmethyl, Tri(methoxyphenyl)methyl, Tri(dimethoxyphenyl)-methyl; Triphenylsilyl, Alkyldiphenylsilyl, Dialkylphenylsilyl und Trialkylsilyl mit 1 bis 20, bevorzugt 1 bis 12 und besonders bevorzugt 1 bis 8 C-Atomen in den Alkylgruppen, zum Beispiel Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; -(C₁-C₈-Alkyl)₂Si-O-Si(C₁-C₈-Alkyl)₂-, worin Alkyl zum Beispiel Methyl, Ethyl n- und i-Propyl, n-, i- oder t-Butyl bedeutet; C₂-C₁₂-, besonders C₂-C₈-Acyl, wie zum Beispiel Acetyl, Propanoyl, Butanoyl, Pentanoyl, Hexanoyl, Benzoyl, Methylbenzoyl, Methoxybenzoyl, Chlorbenzoyl und Brombenzoyl; R_{S1}-SO₂-, worin R_{S1} C₁-C₁₂-Alkyl, besonders C₁-C₆-Alkyl, C₅- oder C₆-Cycloalkyl, Phenyl, Benzyl, C₁-C₁₂- und besonders C₁-C₄-Alkylphenyl, oder C₁-C₁₂- und besonders C₁-C₄-Alkylbenzyl, oder Halogenphenyl oder Halogenbenzyl bedeutet, zum Beispiel Methyl-, Ethyl-, Propyl-, Butyl-, Phenyl-, Benzyl-, p-Brom-, p-Methoxy- und p-Methylphenylsulfonyl; unsubstituiertes oder mit F, Cl, Br, C₁-C₄-Alkoxy, Tri-(C₁-C₄-Alkyl)silyl oder C₁-C₄-Alkylsulfonyl substituiertes C₁-C₁₂-, bevorzugt C₁-C₈-Alkoxycarbonyl, zum Beispiel Methoxy-, Ethoxy-, n-oder i-Propoxy- oder n-, i- oder t-Butoxycarbonyl, 2-Trimethylsilylethoxycarbonyl, 2-Methylsulfonylethoxycarbonyl, Allyloxycarbonyl oder unsubstituiertes oder wie für Alkoxycarbonyl substituiertes Phenyloxycarbonyl oder Benzyloxycarbonyl, zum Beispiel Methyloder Methoxy- oder Chlorphenyloxycarbonyl oder -benzyloxycarbonyl, sowie 9-Fluorenylmethyloxycarbonyl. Sofern R₁ und/oder R₂ Alkyl bedeuten, kann es mit F, Cl, Br, C₁-C₄-Alkoxy, Phenyloxy, Chlorphenyloxy, Methoxyphenyloxy, Benzyloxy, Methoxybenzyloxy oder Chlorphenyloxy substituiert sein. Bei R₁ und R₂ in Formel I kann es sich um gleiche oder verschiedene Schutzgruppen handeln.

In einer besonders bevorzugten Ausführungsform stellen R₁ und R₂ als Schutzgruppen Benzyl, Methylbenzyl, Dimethylbenzyl, Methoxybenzyl, Dimethoxybenzyl, halogeniertes Benzyl, insbesondere Brombenzyl; Diphenylmethyl, Di(methylphenyl)methyl, Di(dimethylphenyl)methyl, Di(methoxyphenyl)methyl, Di(methoxyphenyl)(phenyl)-methyl, Triphenylmethyl, Tris-4,4',4"-tert.butylphenylmethyl, Di-p-anisylphenylmethyl, Tri(methylphenyl)methyl, Tri(dimethylphenyl)methyl, Tri(methoxyphenyl)methyl, Tri(dimethoxyphenyl)methyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl, -(CH₃)₂Si-O-Si(CH₃)₂-, -(i-C₃H₇)₂Si-O-Si(i-C₃H₇)₂-; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl oder 9-Fluorenylmethyloxycarbonyl dar.

R₂ kann als phosphorhaltiger eine Nukleotid-Brückengruppe bildender Rest der Formel P1 oder P2 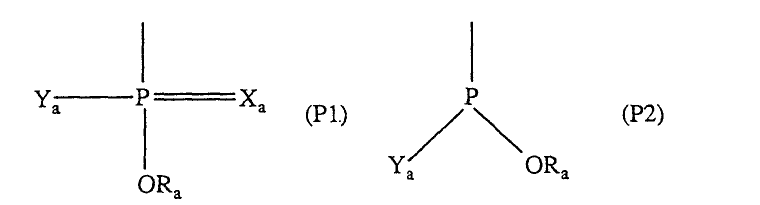 entsprechen, worin Yₐ Wasserstoff, C₁-C₁₂-Alkyl, C₆-C₁₂-Aryl, C₇-C₂₀-Aralkyl, C₇-C₂₀-Alkaryl, -OR_{b}, -SR_{b}, -NH₂, Primäramino, Sekundäramino, O^{⊖}M^{⊕} oder S^{⊖}M^{⊕} darstellt; Xₐ Sauerstoff oder Schwefel bedeutet; Rₐ Wasserstoff, M^{⊕}, C₁-C₁₂-Alkyl, C₂-C₁₂-Alkenyl, C₆-C₁₂-Aryl, oder die Gruppe RₐO- für N-Heteroaryl-N-yl mit 5 Ringgliedern und 1 bis 3 N-Atomen steht; R_{b} Wasserstoff, C₁-C₁₂-Alkyl oder C₆-C₁₂-Aryl bedeutet; und M^{⊕} für Na^{⊕}, K^{⊕}, Li^{⊕}, NH₄^{⊕} steht oder Primär-, Sekundär-, Tertiär- oder Quartenärammonium darstellt; wobei Alkyl, Aryl, Aralkyl und Alkaryl in Yₐ, Rₐ und R_{b} unsubstituiert oder mit Alkoxy, Alkylthio, Halogen, -CN, -NO₂, Phenyl, Nitrophenyl, oder Halogenphenyl substituiert ist.

Yₐ enthält als Primäramino bevorzugt 1 bis 12 und besonders bevorzugt 1 bis 6 C-Atome, und als Sekundäramino bevorzugt 2 bis 12 und besonders bevorzugt 2 bis 6 C-Atome.

Bei dem Primäramino und Sekundäramino kann es sich zum Beispiel um Reste der Formel R_{c}R_{d}N handeln, worin R_{c} für H steht oder unabhängig die Bedeutung von R_{d} hat, und R_{d} C₁-C₂₀-, bevorzugt C₁-C₁₂- und besonders bevorzugt C₁-C₆-Alkyl, C₁-C₂₀-, bevorzugt C₁-C₁₂- und besonders bevorzugt C₁-C₆-Aminoalkyl, C₁-C₂₀-, bevorzugt C₁-C₁₂- und besonders bevorzugt C₁-C₆-Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R_{c} und R_{d} zusammen Tetra- oder Pentamethylen, 3-Oxa-1,5-pentylen, -CH₂-NRₑ-CH₂CH₂- oder -CH₂CH₂-NR₁₉-CH₂CH₂- darstellen, worin Rₑ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Unter Primär-, Sekundär-, Tertiär- und Quartärammonium ist für Yₐ im Zusammenhang mit der Definition von M^{⊕} ein Ion der Formel R_{f}R_{g}RₕRᵢN^{⊕} zu verstehen, bei dem R_{f} C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, und R_{g}, Rₕ und Rᵢ unabhängig voneinander Wasserstoff sind oder die Bedeutung von R_{f} haben, oder R_{f} und R_{g} zusammen Tetra- oder Pentamethylen, 3-Oxa-1,5-pentylen, -CH₂-NRₑ-CH₂CH₂- oder -CH₂CH₂-NRₑ CH₂CH₂darstellen, worin Rₑ für H oder C₁-C₄-Alkyl steht, und Rₕ und Rᵢ unabhängig voneinander die Bedeutung von R_{f} haben. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. R₁₉ stellt bevorzugt H, Methyl oder Ethyl dar.

Bevorzugte Beispiele für Primäramino und Sekundäramino sind Methyl-, Ethyl-, Dimethyl-, Diethyl-, Di-i-Propyl, Mono- oder Di-(1-hydroxy-eth-2-yl)-, Phenyl- und Benzylamino, Acetylamino und Benzoylamino sowie Piperidinyl, Piperazinyl und Morpholinyl.

Bevorzugte Beispiele für Primär- und Sekundärammonium sind Methyl-, Ethyl-, Dimethyl-, Diethyl-, Di-i-Propyl-, Mono- oder Di-(1-hydroxy-eth-2-yl)-, Phenyl- und Benzylammonium.

Beispiele für Yₐ, Rₐ und R_{b} als Alkyl sind Methyl, Ethyl und die Isomeren von Propyl, Butyl, Pentyl, Hexyl, Heptyl und Octyl; Beispiele für Yₐ, Rₐ und R_{b} als Aryl sind Phenyl und Naphthyl; Beispiele für Rₐ als Alkenyl sind Allyl und (C₁-C₄-Alkyl)CH=CH-CH₂-; Beispiele für Yₐ als Aralkyl sind Phenyl-CₙH₂ₙ- mit n gleich einer Zahl von 1 bis 6, besonders Benzyl; Beispiele für Yₐ als Alkaryl sind Mono-, Di- und Tri(C₁-C₄-alkyl)phenyl. Bevorzugte Substituenten sind Chlor, Brom, Methoxy, -NO₂, -CN, 2,4-Dichlorphenyl und 4-NitrophenyL Beispiele für R_{b} sind 2,2,2-Trichlorethyl, 4-Chlorphenyl, 2-Chlorphenyl und 2,4-Dichlorphenyl; und Beispiele für R_{b}O- als N-Heteroaryl sind Pyrrol-N-yl, Triazol-N-yl und Benztriazol-N-yl.

In einer besonders bevorzugten Ausführungsform bedeutet Rₐ β-Cyanoethyl und stellt Yₐ Di(i-propylamino) dar.

Wenn B einen Purinrest oder ein Analoges davon darstellt, so kann es sich um einen Rest der Formel II, IIa, IIb, IIc, IId, IIe oder IIf handeln, 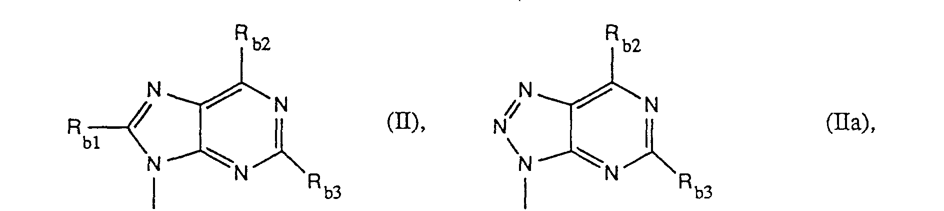 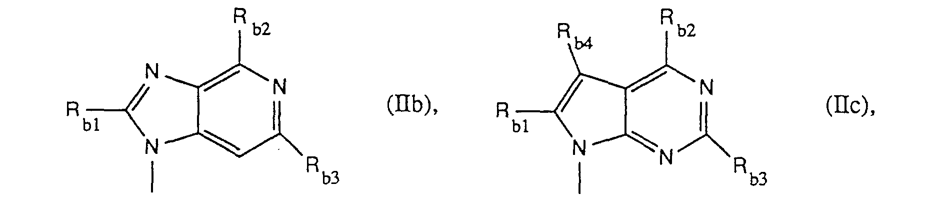 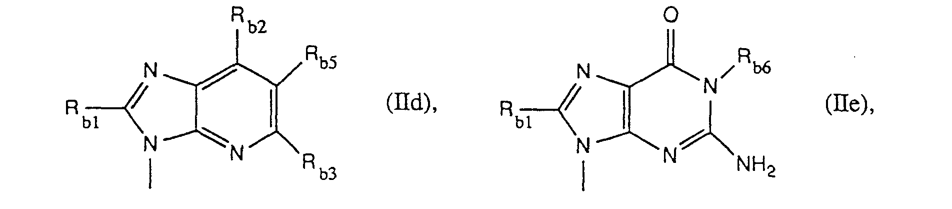 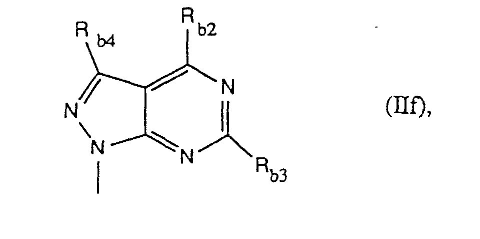 worin R_{b1} für H, Cl, Br, OH oder -O-C₁-C₁₂-Alkyl steht, und R_{b2}, R_{b3} und R_{b5} unabhängig voneinander H, OH, SH, NH₂, NHNH₂, NHOH, NHO-C₁-C₁₂-Alkyl, -N=CH-N(C₁-C₁₂-Alkyl)₂, -N=CH-Azacycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, R_{b4} Wasserstoff, CN oder -C≡C-R_{b7}, R_{b6} und R_{b7} Wasserstoff oder C₁-C₄-Alkyl darstellen.

Geeignete Schutzgruppen sind zuvor erwähnt worden. Bevorzugte Schutzgruppen sind C₁-C₈-Acylgruppen, wie zum Beispiel Acetyl, Propionyl, Butyroyl und Benzoyl. R_{b6} steht bevorzugt für H oder Methyl.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Das Alkyl, Alkoxy, Alkylthio, Hydroxyalkyl und Aminoalkyl enthält besonders bevorzugt 1 bis 4 C-Atome. Bevorzugte Alkyl-, Alkoxy-, Alkylthio-, Hydroxyalkyl- und Aminoalkylreste sind Methyl, Ethyl, n-und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel Rₐ₁Rₐ₂N handeln, worin Rₐ₁ für H steht oder unabhängig die Bedeutung von Rₐ₂ hat, und Rₐ₂ C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder Rₐ₁ und Rₐ₂ zusammen Tetra- oder Pentamethylen, 3-Oxa-1,5-pentylen, -CH₂-NRₐ₃-CH₂CH₂- oder -CH₂CH₂-NRₐ₃-CH₂CH₂- darstellen, worin Rₐ₃ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Alkyl sind zuvor angegeben worden. Beispiele für Aminoalkyl sind Aminomethyl, Aminoethyl, 1-Aminoprop-2-yl oder -3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl- oder N,N-Di-2-hydroxyethylaminomethyl oder -aminoethyl oder -aminopropyl oder -aminobutyl. Beispiele für Hydroxyalkyl sind Hydroxymethyl, 1-Hydroxy-eth-2-yl, 1-Hydroxy-prop-2- oder -3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3-oder 4-en-1-yl oder -2-yl, Hex-3- oder -4- oder -5-en-1-yl oder -2-yl. Beispiele für Alkylund Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. Rₐ₃ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino, Isobutyrylamino und Benzoylamino.

In einer bevorzugten Ausführungsform stellt R_{b1} Wasserstoff dar. In einer anderen bevorzugten Ausführungsform stellt R_{b5} Wasserstoff dar. In einer weiteren bevorzugten Ausführungsform bedeuten R_{b2} und R_{b3} unabhängig voneinander H, F, Cl, Br, OH, SH, NH₂, NHOH, NHNH₂, Methylamino, Dimethylamino, Benzoylamino, Isobutyrylamino, Methoxy, Ethoxy und Methylthio.

Einige Beispiele für Analoge der Purinreihe sind neben Purin Xanthin, Hypoxanthin, Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin, N-Isobutyrylguanin. Besonders bevorzugt sind Adenin, 2-Aminoadenin und Guanin, sowie deren basengeschützte Derivate.

Wenn B in Formel I einen Pyrimidinrest darstellt, so handelt es sich bevorzugt um einen Uracil-, Thymin- oder Cytosinrest der Formel III, IIIa, IIIb oder IIIc 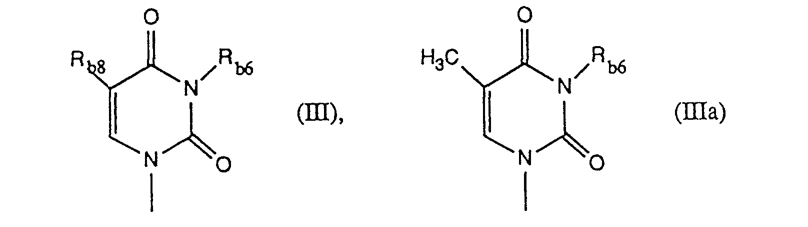 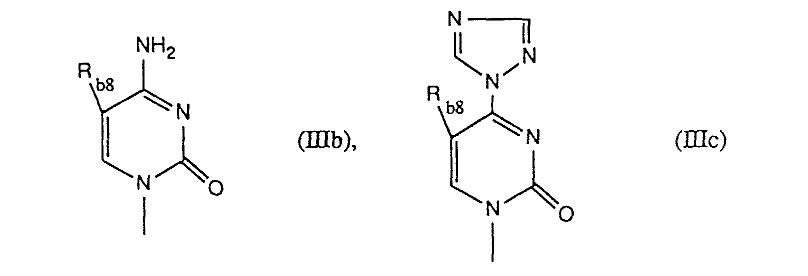 worin R_{b6} H oder C₁-C₄-Alkyl und R_{b8} H, OH, SH, NH₂, NHNH₂, NHOH, NHO-C₁-C₁₂-Alkyl, -N=CH-N(C₁-C₁₂-Alkyl)₂, -N=CH-Azacycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen, Sekundäramino mit 2 bis 30 C-Atomen, C₁-C₁₂-Alkenyl oder C₁-C₁₂-Alkinyl bedeuten, und die NH₂-Gruppe in Formel IIIb unsubstituiert oder mit C₁-C₆-Alkyl, Benzoyl oder einer Schutzgruppe substituiert ist, sowie die Dihydroderivate der Reste der Formeln III, IIIa, IIIb und IIIc. Bevorzugt stellt R_{b8} in Formel III H, C₁-C₆-Alkyl oder -Hydroxyalkyl, C₂-C₆-Alkenyl oder -Alkinyl, F, Cl, Br, NH₂, Benzoylamino, Mono- oder Di-C₁-C₆-alkylamino dar. Bevorzugt stellt R_{b8} in Formel IIIb und IIIc H, C₁-C₆-Alkyl oder -Alkoxy oder -Hydroxyalkyl, C₂-C₆-Alkenyl oder -Alkinyl, F, Cl, Br, NH₂, Benzoylamino, Mono- oder Di-C₁-C₆-alkylamino dar.

R_{b6} steht bevorzugt für H oder Methyl. R_{b8} in Formel III bedeutet bevorzugt H, F, Cl, Br, NH₂, NHCH₃, N(CH₃)₂, C₁-C₄-Alkyl, C₂-C₄-Alkenyl oder C₂-C₄-Alkin-1-yl. R_{b8} in Formel IIIb und IIIc stellt bevorzugt H, C₁-C₄-Alkyl, besonders Methyl, C₂-C₄-Alkenyl, besonders Vinyl oder C₂-C₄-Alkin-1-yl, besonders 1-Propin-1-yl, oder NH₂, NHCH₃ oder (CH₃)₂N dar.

Einige Beispiele für Pyrimidinanaloge sind Uracil, Thymin, Cytosin, 5-Fluoruracil, 5-Chloruracil, 5-Bromuracil, Dihydrouracil, 5-Methylcytosin, 5-Propinthymin und 5-Propincytosin.

Ein weiterer Gegenstand der Erfindung ist ein Verfahren zur Herstellung von Verbindungen der Formel I, 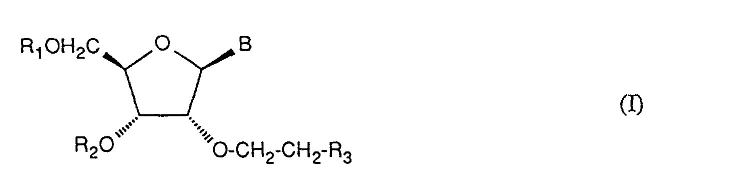 worin R₁ und R₂ unabhängig voneinander für Wasserstoff oder eine Schutzgruppe stehen; und B einen Purin- oder Pyrimidinrest oder ein Analoges davon bedeutet; und
(a) R₃ OH bedeutet,
   das dadurch gekennzeichnet ist, dass man eine Verbindung der Formel IVa 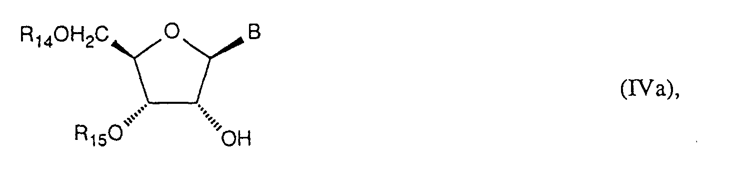 worin R₁₄ und R₁₅ für gleiche oder verschiedene Schutzgruppen stehen und B einen Purinoder Pyrimidinrest oder ein Analoges davon bedeutet, wobei funktionelle Gruppen im Basenrest B durch Schutzgruppen geschützt sind, in einem inerten Lösungsmittel mit einer Verbindung der Formel A umsetzt

   X-CH₂-COOR₄ (A),

   worin R₄ C₁-C₄-Alkyl und X Cl, Br, I, Tosyl-O oder Mesyl-O bedeutet;
   und die Esterfunktion anschliessend mit NaBH₄ oder LiAlH₄ reduziert, wobei die entstehende OH-Gruppe vorübergehend mit einer Gruppe wie für R₁ definiert geschützt werden kann;
(b) R₃ F bedeutet,
   das dadurch gekennzeichnet ist, dass man eine Verbindung der Formel I, worin R₃ OH bedeutet, mit einer Verbindung der Formel B umsetzt 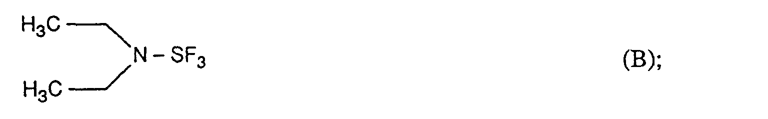
(c) R₃ -(CF₂)ₙ-CF₃ bedeutet, worin n für eine Zahl von 0 bis 7 steht,
   das dadurch gekennzeichnet ist, dass man eine Verbindung der Formel IVa mit einer Verbindung der Formel C, D oder E umsetzt

   CH≡C-(CF₂)ₙ-CF₃ (C)

   CH₂=CH-(CF₂)ₙ-CF₃ (D)

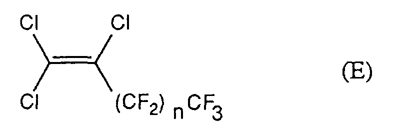

   und anschliessend die gegebenenfalls vorhandene Doppelbindung oder chlorierte Doppelbindung katalytisch zu CH₂CH₂(CF)ₙCF₃ reduziert;
(d) R₃ OH, F oder -(CF₂)ₙ-CF₃ bedeutet,
   das dadurch gekennzeichnet ist, dass man eine Verbindung der Formel IVb 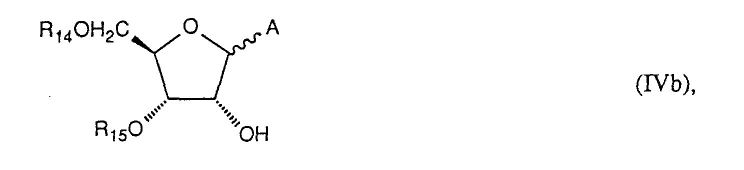 worin R₁₄ und R₁₅ die oben erwähnte Bedeutung haben und A für eine Abgangsgruppe, bevorzugt Alkoxy, Acyloxy, Mesyl-O, Tosyl-O und besonders bevorzugt OCH₃, OCOCH₃ und Benzoyloxy steht, nach einem der in (a) bis (c) beschriebenen Verfahren an der 2'-OH Gruppe substituiert und dann in an sich bekannter Weise den Basenrest B durch Substitution einführt [E. Lukevics, A. Zablocka, Nucleoside Synthesis, Ellis Horwood, New York (1991)]; und gegebenenfalls die Schutzgruppen R₁₄ und R₁₅ abspaltet.

Die Verbindungen der Formeln IVa und IVb, A, B, C, D und E sind bekannt, teilweise käuflich oder nach bekannten beziehungsweise analogen Verfahren herstellbar.

Inerte Lösungsmittel sind zum Beispiel Kohlenwasserstoffe, Halogenkohlenwasserstoffe, alkylierte Carbonsäureamide und Lactame, Ether, Nitrile wie Acetonitril, Dialkylsulfone oder -sulfoxide oder cyclische Sulfone und Sulfoxide.

Die Reaktionstemperaturen bei den Verfahrensstufen (a) bis (d) liegen zwischen -50 bis 200°C, bevorzugt zwischen 0 bis 90°C.

Die Umsetzungen werden ausser mit B vorteilhaft in Gegenwart von Basen durchgeführt, zum Beispiel Alkalimetallhydriden, -alkoholaten, -hydroxiden, -carbonaten, Trialkylaminen oder Diazabicycloundecen.

Die Isolierung der Verbindungen der Formel I und deren Reinigung erfolgt nach an sich bekannten Verfahren wie zum Beispiel Fällung oder Kristallisation und Filtration und chromatographischen Methoden.

Aus den Verbindungen der Formel I können Oligonukleotide aufgebaut werden, die auf Grund ihrer Wechselwirkung mit Nukleinsäuren wertvolle biologische Aktivitäten aufweisen, und als pharmazeutische Wirkstoffe oder als Diagnostika verwendet werden können.

Ein weiterer Gegenstand der Erfindung ist die Verwendung der Verbindungen der Formel I zur Herstellung von Oligonukleotiden, die gleiche oder verschiedene Monomereinheiten von Verbindungen der Formel I enthalten, jedoch mindestens eine Monomereinheit von Verbindungen der Formel I in Kombination mit Monomereinheiten von anderen natürlichen oder synthetischen Nukleosiden, wobei die Oligonukleotide 2 bis 200 Monomereinheiten enthalten. Bevorzugt enthalten die Oligonukleotide 2 bis 100, besonders bevorzugt 2 bis 50, und insbesondere bevorzugt 4 bis 30 Monomereinheiten. Bevorzugt sind Oligonukleotide, die gleiche oder verschiedene Monomereinheiten von Verbindungen der Formel I enthalten. Bevorzugt sind ferner Oligonukleotide, die zusätzlich Monomerein heiten von synthetischen oder natürlichen Nukleosiden enthalten, die sich von D-Ribose oder 2-Desoxyribose ableiten.

Ein weiterer Gegenstand der Erfindung sind Oligonukleotide der Formel V

5'-U-(O-Y-O-V-)ₓO-Y-O-W-3' (V),

worin x eine Zahl von 0 bis 200 und Y eine Nukleotid-Brückengruppe bedeuten, U, V und W je für sich gleiche oder verschiedene Reste von natürlichen oder synthetischen Nukleosiden darstellen und mindestens einer der Reste U, V und/oder W einen Rest der Formel VI 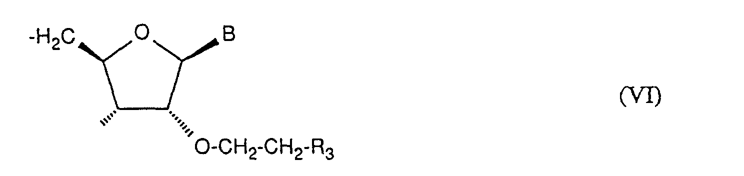 bedeutet, und B und R₃ die für die Verbindungen der Formel I angegebenen Bedeutungen haben einschliesslich der Bevorzugungen und Beispiele.

Eine bevorzugte Brückengruppe Y ist die in natürlichen Oligonukleotiden vorkommende Gruppe -P(O)O^{⊖}-. Beispiele für weitere Brückengruppen sind -P(O)S^{⊖}-, -P(S)S^{⊖}-, -P(O)R₁₆-, P(O)NR₁₇R₁₈, oder -CH₂-, worin R₁₆ H oder C₁-C₆-Alkyl darstellt und R₁₇ und R₁₈ unabhängig voneinander die Bedeutung von R₁₆ haben. In Formel V steht x bevorzugt für eine Zahl von 0 bis 100, besonders bevorzugt für eine Zahl von 1 bis 50 und insbesondere bevorzugt für eine Zahl von 3 bis 29. Die Reste der Formel VI können endständig oder in der Nukleotidsequenz gebunden sein, wobei alle oder mehrere, zum Beispiel 2 bis 5 Reste der Formel VI aufeinander folgen können, oder die Reste der Formel VI können zwischen Resten von natürlichen oder synthetischen Nukleosiden gebunden sein, oder es können Mischformen dieser Verteilungen in der Nukleotidsequenz vorliegen.

Eine ganz besonders bevorzugte Ausführungsform sind Oligonukleotide der Formel V, worin x eine Zahl von 2 bis 50, bevorzugt 2 bis 30 darstellt, Y für die Gruppe -P(O)O^{⊖}steht, U, V und W je für sich gleiche oder verschiedene Reste eines natürlichen Nukleosids bedeuten und mindestens einer der Reste U, V oder W der Formel VI entspricht. Als natürliche Nukleoside kommen Adenosin, Cytidin, Guanosin, Uridin, 2-Aminoadenin, 5-Methylcytosin, 2'-Desoxyadenosin, 2'-Desoxycytidin, 2'-Desoxyguanosin und Thymidin in Frage. Als natürliche Nukleosidbasen sind besonders Adenin, Cytosin, Guanin, Thymin und Uracil zu nennen. Die Reste der Formel VI können endständig oder in der Nukleotidsequenz gebunden sein, wobei alle oder mehrere, zum Beispiel 2 bis 5 gleiche oder verschiedene Reste der Formel VI aufeinander folgen können, oder gleiche oder verschiedene Reste der Formel VI zwischen Resten von natürlichen Nukleosiden gebunden sind, oder Mischformen dieser Verteilungen in der Nukleotidsequenz vorliegen. In einer anderen bevorzugten Ausführungsform von Oligonukleotiden der Formel V entsprechen sämtliche Reste U, V und W gleichen oder verschiedenen Resten der Formel VI. Bevorzugt stellt x eine Zahl von 3 bis 29 dar und bevorzugt sind insgesamt 1 bis 12 Reste der Formel VI enthalten.

Die Herstellung der erfindungsgemässen Oligonukleotide kann in an sich bekannter Weise nach verschiedenen Verfahren in gegebenenfalls automatisierten und zusammen mit Verfahrensvorschriften käuflichen DNA-Synthesizem erfolgen. Im Falle der Brückengruppe -P(O)O^{⊖}- kann zum Beispiel das Phosphortriesterverfahren, das Phosphittriesterverfahren oder das H-Phosphonatverfahren angewendet werden, die dem Fachmann geläufig sind. Beim Phosphittriesterverfahren kann man zum Beispiel so vorgehen, dass man die Nukleoside der Formel I, worin R₁ und R₂ je H bedeuten, mit einem Schutzgruppenreagenz, zum Beispiel 4,4'-Dimethoxytriphenylmethyl-chlorid zu einem Nukleosid der Formel F umsetzt 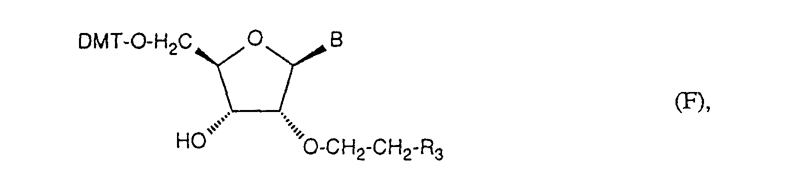 und die Verbindung der Formel F mit Hilfe eines "linkers", zum Beispiel Bemsteinsäureanhydrid, an ein festes Trägermaterial bindet, zum Beispiel an Controlled Pore Glass (CPG), das langkettige Alkylaminogruppen enthält. In einem separaten Verfahren wird die Hydroxylgruppe der Verbindung der Formel F derivatisiert, zum Beispiel zu einem Phosphoramidit unter Verwendung von R'OP[N(i-Propyl)₂)]₂ zu einer Verbindung der Formel G 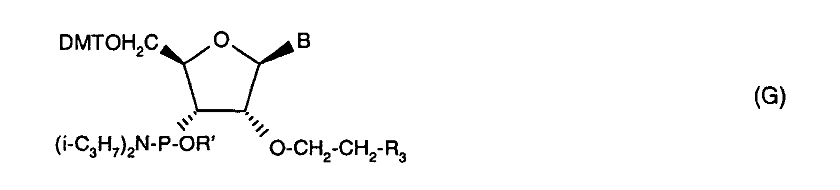 wobei R' zum Beispiel β-Cyanoethyl darstellt.

Nach dem Abspalten der Schutzgruppe wie zum Beispiel der DMT-Gruppe des an den Träger gebundenen Materials koppelt man unter Abspaltung von -N(i-C₃H₇)₂ mit der Verbindung der Formel F, blockiert eventuell vorhandene freie Hydroxylgruppen (capping) und oxidiert dann das gebildete Phosphit zum Phosphat. Nach dem Entschützen des Dimeren wiederholt man den Reaktionszyklus mit einer Verbindung der Formel G, bis man ein Oligomer mit der gewünschten Anzahl an Monomereinheiten synthetisiert hat, und löst das Produkt vom Trägermaterial ab. Auf diese Weise erhält man Oligonukleotide, in denen sämtliche Reste U, V und W gemäss Formel V aus Resten der Formel VI bestehen. Auf diese Weise sind auch Oligonukleotide mit beliebigen Monomereinheiten in beliebiger Sequenz herstellbar, je nach Verwendung synthetischer, natürlicher und erfindungsgemässer Nukleosidbausteine in den einzelnen Reaktionszyklen.

Die erfindungsgemässen Verbindungen der Formel I, worin R₁ und R₂ je H bedeuten, weisen antivirale und antiproliferative Eigenschaften auf und können demgemäss als Arzneimittel Verwendung finden. Die erfindungsgemässen Oligonukleotide weisen zudem eine hohe Stabilität gegenüber einem Abbau durch Nukleasen auf. Besonders überraschend ist ihre ausgezeichnete Paarung mit komplementären Nukleinsäuresträngen, vor allem vom RNA-Typ. Zusätzlich zeigen sie eine unerwartet hohe zelluläre Aufnahme. Die erfindungsgemässen Oligonukleotide eignen sich daher besonders für die Antisense-Technologie, das heisst zur Inhibition der Expression unerwünschter Proteinprodukte durch die Bindung an geeignete komplementäre Nukleotidsequenzen von mRNA (EP266,099, WO87/07300 und WO89/08146). Sie können zur Behandlung von Infektionen und Krankheiten zum Beispiel durch die Blockierung der Expression von bioaktiven Proteinen auf der Stufe der Nukleinsäuren (zum Beispiel Onkogene) eingesetzt werden. Die erfindungsgemässen Oligonukleotide eignen sich auch als Diagnostika und können als Gensonden zum Nachweis von viralen Infektionen oder genetisch bedingten Krankheiten durch selektive Interaktion auf der Stufe von einzel- oder doppelsträngigen Nukleinsäuren verwendet werden ("gene probes"). Im besonderen - bedingt durch die erhöhte Stabilität gegenüber Nukleasen - ist eine diagnostische Anwendung in vitro (zum Beispiel an isolierten Gewebeproben, Blutplasma und Blutserum) möglich. Solche Verwendungsmöglichkeiten sind zum Beispiel in der WO 91/06556 beschrieben.

Ein weiterer Gegenstand der Erfindung betrifft die Verwendung der erfindungsgemässen Oligonukleotide als Diagnostika zum in vitro Nachweis von viralen Infektionen oder genetisch bedingten Krankheiten.

Ein weiterer Gegenstand der Erfindung betrifft ein pharmazeutisches Präparat, enthaltend eine wirksame Menge eines Nukleosids der Formeln I oder eines Oligonukleotids der Formeln V alleine oder zusammen mit anderen Wirkstoffen, ein pharmazeutisches Trägermaterial vorzugsweise in einer signifikanten Menge und gegebenenfalls Hilfsstoffe.

Man kann die pharmakologisch wirksamen erfindungsgemässen Nukleoside und Oligonukleotide in Form von parenteral verabreichbaren Präparaten oder von Infusionslösungen verwenden. Solche Lösungen sind vorzugsweise isotonische wässrige Lösungen oder Suspensionen, wobei diese zum Beispiel bei lyophilisierten Präparaten, welche die Wirksubstanz allein oder zusammen mit einem Trägermaterial, zum Beispiel Mannit, enthalten, vor Gebrauch hergestellt werden können. Die pharmazeutischen Präparate können sterilisiert sein und/oder Hilfsstoffe, zum Beispiel Konservier- Stabilisier-, Netz- und/oder Emulgiermittel, Löslichkeitsvermittler, Salze zur Regulierung des osmotischen Drucks und/oder Puffer enthalten. Die pharmazeutischen Präparate, die gewünschtenfalls weitere pharmakologisch wirksame Stoffe wie zum Beispiel Antibiotka enthalten können, werden in an sich bekannter Weise, zum Beispiel mittels konventioneller Lösungs- oder Lyophilisierungsverfahren, hergestellt, und enthalten etwa 0,1 % bis 90 %, insbesondere von etwa 0,5 % bis etwa 30 %, zum Beispiel 1 % bis 5 % Aktivstoff(e).

Die nachfolgenden Beispiele erläutern die Erfindung. Den ¹H-NMR-Spektren liegt die Nummerierung der Kohlenstoffatome in den folgenden cyclischen Kohlenstoffgerüsten zu Grunde:
Ausgangsverbindungen: 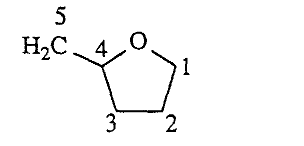
Nukleoside (Beispiele):

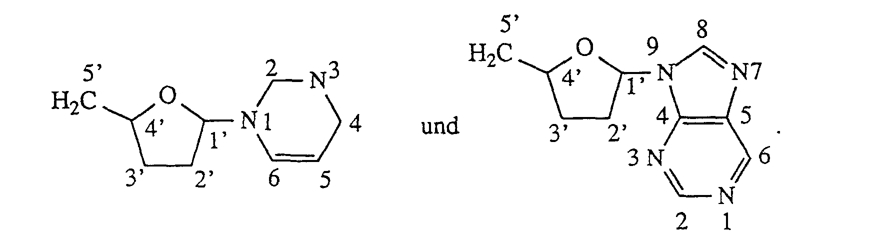

Verwendete Abkürzungen im Text und in den Formeln:
- DMF: Dimethylformamid
- ClBnCl₂: 2,4-Dichlorbenzylchlorid
- Bn: Benzyl
- Ac: Acetyl
- φ: Phenyl
- BSA: N,N-Bistrimethylsilylacetamid
- DBU: Diazabicyclo[5.4.0]undec-7-en
- BOM-Cl: Benzyloxymethylchlorid
- DMTCl: 4,4'-Dimethoxytritylchlorid
- THF: Tetrahydrofuran

### A) Herstellung von Nukleosid-Analogen

### Beispiel A1:

Zu einer Vorlage von 13,5 g NaH in 130 ml DMF werden bei 60°C 28,0 g 1-Methylribose zugetropft. Nach Ende der H₂-Entwicklung werden 110,0 g ClBnCl₂ zugetropft. Das Reaktionsgemisch wird 16 Stunden bei 25°C weitergerührt. Um noch vorhandenes NaH zu zerstören tropft man vorsichtig Methanol zu und giesst das Reaktionsgemisch dann auf Eis/Wasser. Der klumpige Niederschlag wird abfiltriert und mit Acetonitril gut nachgewaschen. Man erhält die Verbindung (A1). 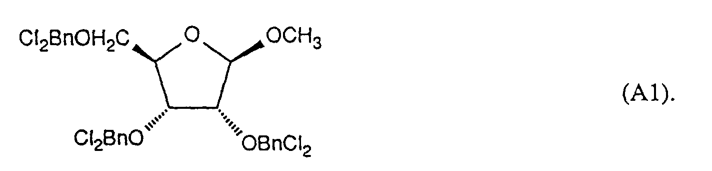 ¹H-NMR (250 MHz, CDCl₃): Das H-C(l)-Proton erscheint bei 5,0 ppm als Singulett.
MS: 638 (M⁺)

### Beispiel A2:

65,9 g des in Beispiel A1 hergestellten Produktes werden in 600 ml Methylenchlorid gelöst und auf 0°C gekühlt. Dann tropft man 121 ml SnCl₄ in 800 ml Methylenchlorid zu und lässt bei 3°C stehen. Nach 26 Stunden werden nochmals 2 ml SnCl₄ zugegeben. Nach total 35 Stunden wird die Reaktionslösung vorsichtig auf 700 ml einer gesättigten NaHCO₃-Lösung gegossen. Nach Verdünnung mit 400 ml Methylenchlorid wird der Sn-haltige Niederschlag abfiltriert. Die organische Phase des Filtrats wird mit MgSO₄ getrocknet und zur Verbindung (A2) eingedampft. 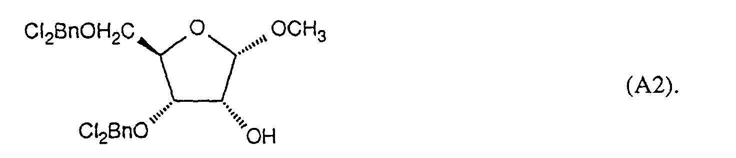 ¹H-NMR (250 MHz, CDCl₃): Das H-C(1)-Proton erscheint bei 4,90 ppm als Doublett mit J=5 Hz.

### Beispiel A3:

125,9 g des in Beispiel A2 erhaltenen Produktes werden in 1 l Pyridin gelöst. Bei 20°C werden 25,5 g Acetanhydrid und 1 g 4-Dimethylaminopyridin zugegeben. Anschliessend wird 17 Stunden gerührt. Das Reaktionsgemisch wird in 1 l Wasser aufgenommen, mit konzentrierter Salzsäure angesäuert und mit Essigsäureethylester extrahiert. Der Extrakt wird mit MgSO₄ getrocknet und eingedampft. Schliesslich wird der Rückstand mit Hexan zur Kristallisation gebracht. Man erhält die Verbindung (A3). 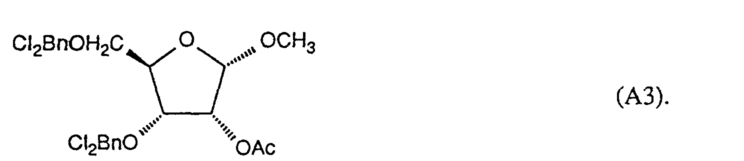 ¹H-NMR (250 MHz, CDCl₃): 5,15 [d, J=4,5 Hz, H-C(1)]; 3,50 (s, OCH₃); 2,17 (s, OCOCH₃);
MS: 522 (M⁺)
[α]_{Na(D)}=87,4±1,0°, CHCl₃ (0,998%)

### Beispiel A4:

24 g Thymin werden in 100 ml 1,2-Dichlorethan aufgeschlämmt. Nach Zugabe von 116,4 g BSA wird unter Rückfluss erhitzt bis eine klare Lösung entsteht. Danach wird auf 50°C abgekühlt und es werden 50 g des in Beispiel A3 hergestellten Produktes sowie 27,5 g Trifluormethansulfonsäuretrimethylsilylester zugegeben. Es wird während 20 Stunden bei 70°C gerührt und anschliessend auf 300 ml NaHCO₃-Lösung gegossen und filtriert. Nach Extraktion mit Dichlorethan wird mit MgSO₄ getrocknet und eingedampft. Schliesslich wird der Rückstand mit Methanol zur Kristallisation gebracht. Man erhält die Verbindung (A4). 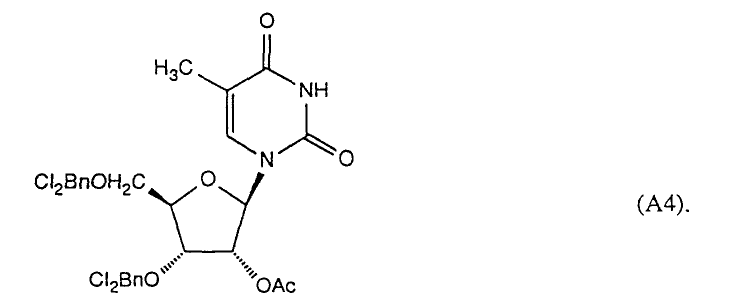 ¹H-NMR (250 MHz, CDCl₃): 8,25 (s, NH); 6,10 [d, J=4,5 Hz, H-C(1')]; 2,13 (s,OCOCH₃); 1,66 (s,CH₃)
MS: 616 (M⁺)

### Beispiel A5:

85 g des in Beispiel A4 hergestellten Produktes werden in 850 ml Acetonitril suspendiert. Es werden bei Raumtemperatur 24,2 g DBU und 24,9 g BOM-Cl zugetropft. Nach 20 Stunden Rühren wird das Reaktionsgemisch auf Wasser gegossen und mit Essigsäureethylester extrahiert. Der Extrakt wird mit MgSO₄ getrocknet und eingedampft. Man erhält die Verbindung (A5). 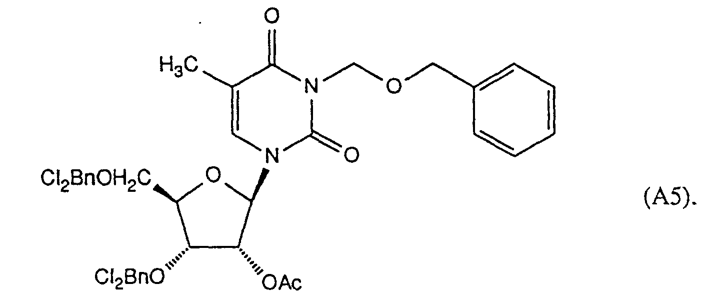 ¹H-NMR (250 MHz, CDCl₃): 6,05 [d, J=4,5 Hz, H-C(1')]; 5,5 (AB, CH₂); 5,37 [dd, H-C(2')]; 2,13 (s, OCOCH₃); 1,55 (s, CH₃)
MS: 736 (M⁺)

### Beispiel A6:

106 g des in Beispiel A5 hergestellten Produktes werden in 1 l THF suspendiert. Es werden 26 g einer 30 %igen NaOCH₃/CH₃OH-Lösung zugetropft. Nach 2,5 Stunden Rühren wird die Reaktionslösung auf Wasser gegossen, mit gesättigter wässriger Kochsalzlösung versetzt und mit Essigsäureethylester extrahiert. Nach Trocknung mit MgSO₄ wird der Extrakt eingedampft. Man erhält die Verbindung (A6). 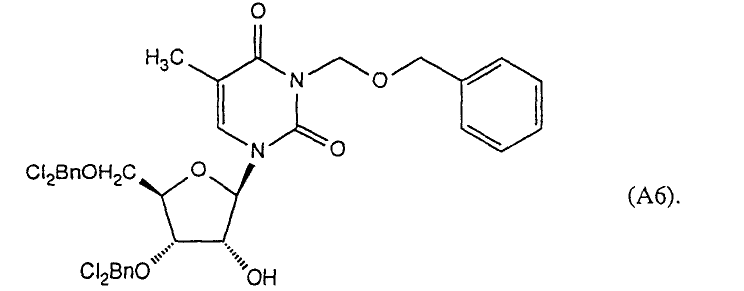 ¹H-NMR (250 MHz, CDCl₃): 5,93 [d, J=5 Hz, H-C(1')]; 5,5 (AB, CH₂); 3,03 (d, J=6,5 Hz, OH); 1,72 (s, CH₃)
MS: 694 (M⁺)

### Beispiel A7:

79,4 g des in Beispiel A6 erhaltenen Produktes werden in 800 ml THF gelöst. Nach Zugabe von 3,3 g NaH wird kurz aufgekocht und anschliessend werden bei 40°C 21 g Bromessigsäuremethylester zugetropft. Das Reaktionsgemisch wird insgesamt 27 Stunden bei 60°C gerührt, wobei nach 16 Stunden und nach 20 Stunden je 1 g NaH und 2 ml Bromessigsäuremethylester zugegeben werden. Schliesslich wird das Reaktionsgemisch auf Wasser gegossen und mit Essigsäureethylester extrahiert. Der Extrakt wird mit MgSO₄ getrocknet und eingedampft. Dabei erhält man die Verbindung (A7). 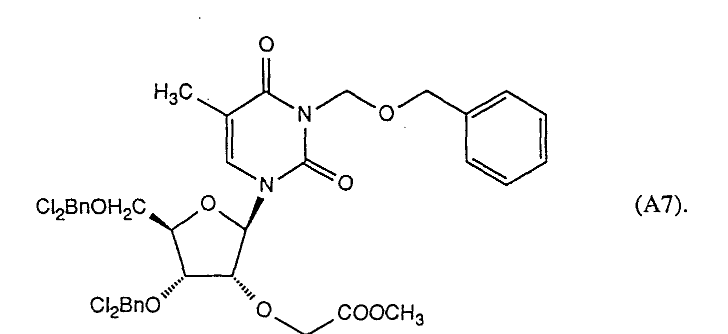 ¹H-NMR (250 MHz, CDCl₃): 7,70 [s, H-C(6)]; 5,92 [s, H-C(1')]; 5,48 (AB, CH₂); 3,75 (s, OCH₃); 1,58 (s, CH₃).
MS: 766 (M⁺).

### Beispiel A8:

37 g des gemäss Beispiel A7 gewonnenen Produktes werden in 400 ml THF gelöst. Man gibt bei 20°C portionsweise 1,5 g LiBH₄ zu und rührt 1 Stunde lang. Danach giesst man das Reaktionsgemisch vorsichtig auf 500 ml Wasser und neutralisiert mit 32 ml 2 N wässriger Salzsäure. Nach Extraktion mit Essigsäureethylester und Eindampfen erhält man die Verbindung (A8). 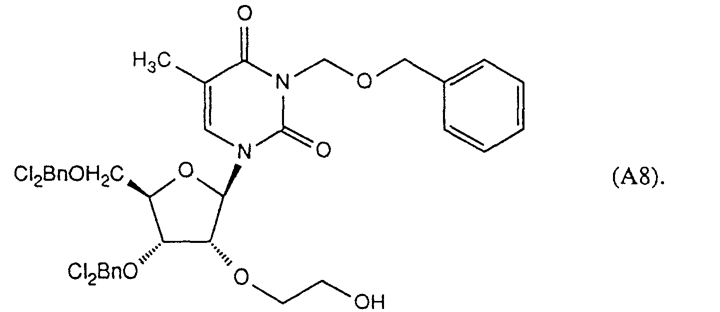 ¹H-NMR (250 MHz, CDCl₃): 7,65 [s, H-C(6)]; 5,96 [s, H-C(1')]; 5,50 (AB, CH₂); 2,57 (breites s, OH); 1,60 (s, CH₃).
MS: 738 (M⁺).

### Beispiel A9:

20,0 g des in Beispiel A8 hergestellten Produktes werden in 200 ml THF gelöst und über 2 g Pd/C (5%) bei 25°C und unter Normaldruck 4,5 Stunden hydriert (H₂-Aufnahme 102 %). Nach Filtration und Eindampfen des Filtrats wird der Rückstand in 170 ml Methanol gelöst und mit einer 30%igen NaOCH₃/CH₃OH-Lösung auf einen pH-Wert von 11 eingestellt. Nach 24 Stunden giesst man auf 250 ml Wasser, säuert mit 2 N wässriger Salzsäure an und extrahiert mit Essigsäureethylester. Der Extrakt wird mit MgSO₄ getrocknet und eingedampft. Man erhält die Verbindung (A9). 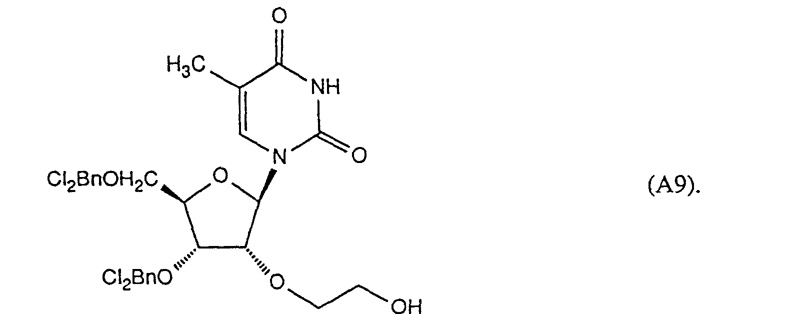 ¹H-NMR (250 MHz, CDCl₃): 9,24 (s, NH); 7,90 [s, H-C(6)]; 5,99 [s, H-C(1')]; 2,68 (t, OH); 1,60 (s, CH₃).
MS: 618 (M⁺).

### Beispiel A10:

4,2 g des in Beispiel A9 hergestellten Produktes werden in 50 ml Pyridin gelöst und nach Zugabe von 2,4 g Acetanhydrid wird 19 Stunden bei Raumtemperatur gerührt. Die Lösung wird auf 100 ml 2 N HCI gegossen und mit Essigsäureethylester extrahiert. Der Extrakt wird mit 2 N HCl und Wasser gewaschen, über MgSO₄ getrocknet und eingedampft. Man erhält die Verbindung (A10). 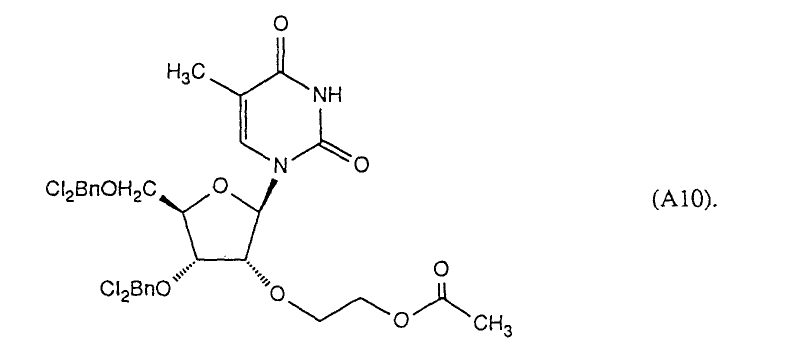 ¹H-NMR (250 MHz, CDCl₃): 9,28 (s, NH); 7,67 [s, H-C(6)]; 5,95 [s, H-C(1')]; 2,00 (s, CH₃); 1,60 (s, CH₃).
MS: 663 (M+H)⁺.

### Beispiel A11:

4,2 g des in Beispiel A10 hergestellten Produktes werden in 50 ml Methanol in Gegenwart von 2,09 g wasserfreiem Natriumacetat über 0,8 g Pd/C (5%) bei 35°C und unter Normaldruck hydriert. Nach 58 Stunden wird das Hydriergemisch filtriert und eingedampft. Der Rückstand wird zur Entfernung von Salzen über eine kleine Fritte mit Kieselgel chromatographiert (Essigsäureethylester/Methanol 9:1). Man erhält die Verbindung (A11). 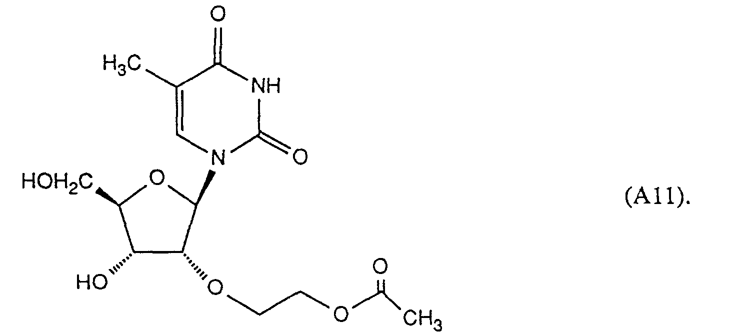 ¹H-NMR (250 MHz, DMSO): 10,1 (s, NH); 7,61 [s, H-C(6)]; 5,64 [d, J=6 Hz, H-C(1')]; 1,70 (s, CH₃); 1,57.
MS: 379 (M+Cl)⁻.

### Beispiel A12:

2,27 g des in Beispiel A11 hergestellten Produktes werden zweimal in Pyridin aufgenommen und eingedampft. Man nimmt erneut in 30 ml Pyridin auf und gibt 2,57 g DMTCl dazu. Nach 20 Stunden Rühren bei Raumtemperatur wird das Reaktionsgemisch mit 250 ml Essigsäureethylester verdünnt und auf 50 ml Wasser gegossen. Die organische Phase wird mit MgSO₄ getrocknet und eingeengt. Den Rückstand chromatographiert man über Kieselgel (Toluol/Essigsäureethylester/Triethylamin 49:49:2). Man erhält die Verbindung (A12). 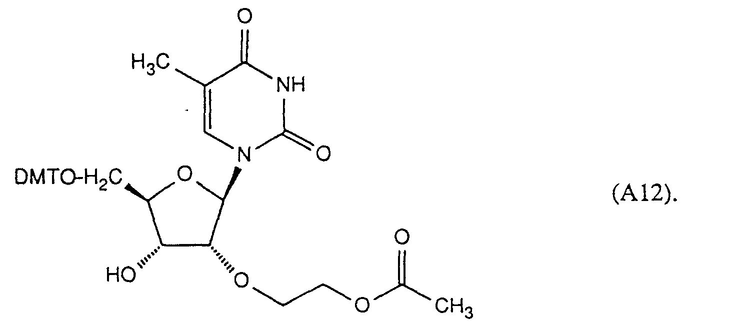 ¹H-NMR (250 MHz, CDCl₃): 9,22 (s,NH); 7,70 [s, H-C(6)]; 5,94 [d, J=1,5 Hz, H-C(1')]; 3.78 (s, OCH₃); 2,19 (s, CH₃); 1,36 (s, CH₃).
MS: 706 (M+NH₄)⁺.

### Beispiel A13:

2,70 g des in Beispiel A12 erhaltenen Produktes werden zu einer Vorlage aus 0,93 g Düsopropylammoniumtetrazolid, 1,51 g 2-Cyanoethyl-N,N,N',N'-tetraisopropylphosphordiamidit und 30 ml Methylenchlorid gegeben. Das Reaktionsgemisch wird 17 Stunden bei Raumtemperatur gerührt und danach auf eine gesättigte wässrige NaHCO₃-Lösung gegossen. Die organische Phase wird mit MgSO₄ getrocknet und eingedampft. Den Rückstand chromatographiert man über Kieselgel (Ethanol/Essigsäureethylester 1:1 mit 2 % Zusatz von Triethylamin). Der erhaltenen Schaum wird in 1 ml Methyl-t-butylether gelöst und bei 0°C in Pentan getropft. Man erhält die Verbindung (A13) (Diastereoisomere, 1:1). 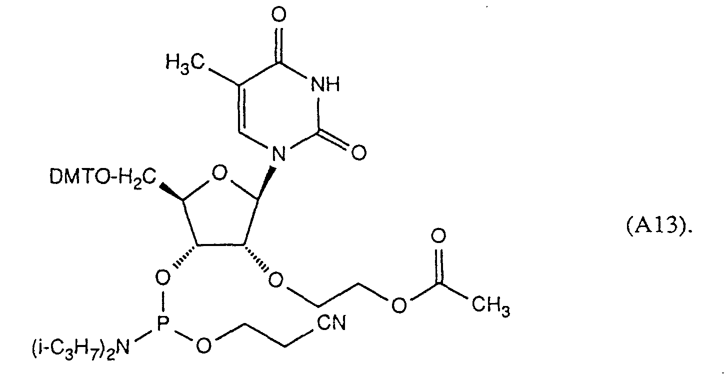 ¹H-NMR (250 MHz, CDCl₃): 7,74 [s, H-C(6)] und 7,68 [s, H-C(6)]; 6,08 [d, J=4 Hz, H-C(1)]; 5,97 [d, J=4Hz, H-C(1')]; ³¹P-NMR(CDCl₃): 150,174 und 150,038
MS: 847 (M+H)⁺

### Beispiel 14:

20,6 g des in Beispiel A8 erhaltenen Produktes werden in 200 ml CH₂Cl₂ gelöst. Bei 5°C werden 4,35 g Diethylamino-schwefeltrifluorid (DAST) zugetropft und das Reaktionsgemisch 3 Stunden bei 5°C gerührt. Danach giesst man die Lösungauf 300 ml gesättigte NaHCO₃-Lösung und extrahiert mit CH₂Cl₂. Der Extrakt wird über MgSO₄ getrocknet und eingedampft. Der Rückstand wird chromatographiert (Kieselgel, Toluol/Essigsäureethylester 1:1). Man erhält die Verbindung (A14). 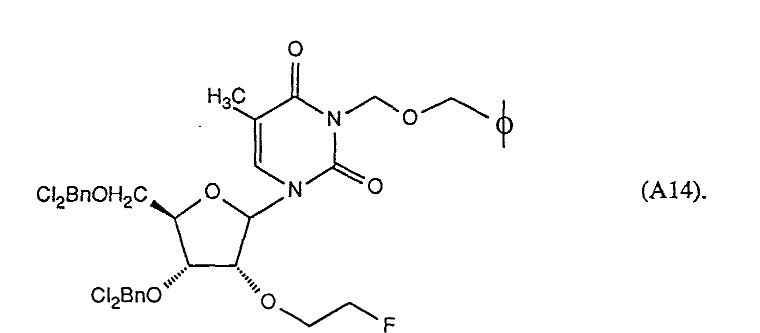 ¹H-NMR (250 MHz, CDCl₃): 7,68 [s, H-C(6)]; 5,92 [s, H-C(1')]; 5,50 [AB, CH₂] 1,58 (s, CH₃). ¹⁹F-NMR (CDCl₃): -223,74 (t, CH₂F).
MS: 758 (M+NH₄)⁺.

### Beispiel A15:

1,30 g des in Beispiel A14 erhaltenen Produktes werden in 26 ml THF gelöst und über 0,65 g Pd/C (5 %) bei 20°C und Normaldruck hydriert. Nach 0,5 Stunden wird der Katalysator abfiltriert und das Filtrat eingedampft. Der Rückstand wird in Methanol (15 ml) aufgenommen und mit NaOMe/MeOH-Lösung auf pH 11 eingestellt. Nach 20 Stunden Rühren wird auf 20 ml Wasser gegossen und mit Essigsäuremethylester extrahiert. Nach dem Eindampfen erhält man die Verbindung (A15). 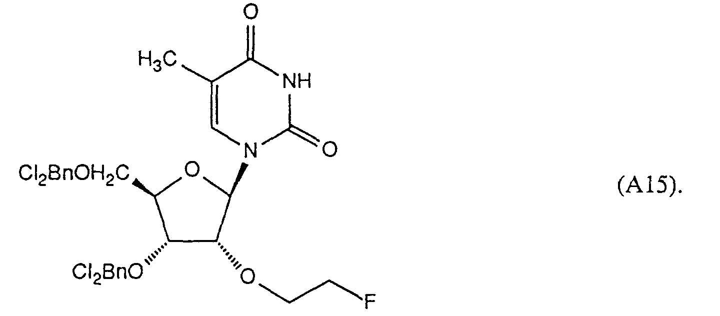 ¹H-NMR (250 MHz, CDCl₃): 8,99 (s, NH); 7,61 [s, H-C(6)]; 5,88 [d, J=1,5Hz, H-C(1')]; 1,52 [s, CH₃].
MS: 621 (M+H)⁺.

### Beispiel A16:

Analog den Arbeitsvorschriften (A11), (A12) und (A13) wird die Verbindung (A15) in das Phosphoramidit (A16) (Diastereoisomere, 1:1) überführt. 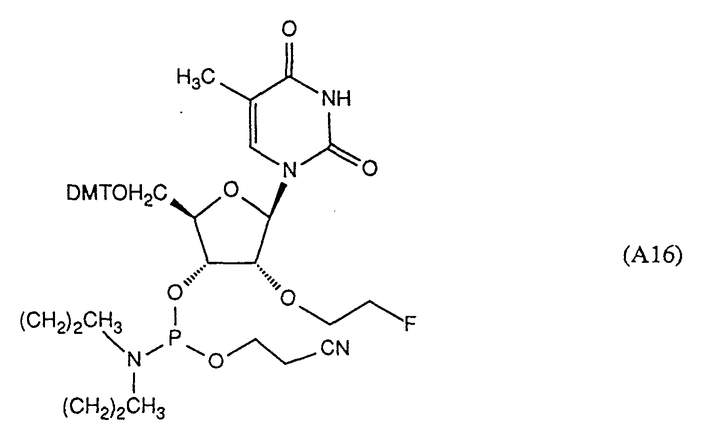 ¹H-NMR (250 MHz, cDCl₃): 7,75 [s, H-C(6)] und 7,68 [s, H-C(6)]; 6,06 [d,J=4 Hz, H-C(1')] und 6,00 [d, J= 4 Hz, H-C(1')].
³¹P-NMR (CDCl₃): 150,107.

### Beispiel A17:

20,0 g der Verbindung (A6) werden in 200 ml THF gelöst und mit 0,83 g NaH (100 %) bis zur Beendigung der H₂-Entwicklung auf 60°C gehalten. Nach dem Abkühlen wird zu dieser Lösung im Autoklaven 48,0 g Trifluorpropin aufgepresst und auf 50°C erwärmt. Nach 48 Stunden wird das Reaktionsgemisch zur Hälfte aufkonzentriert, dann auf Wasser gegossen und mit Essigsäureethylester extrahiert. Der Extrakt wird getrocknet (MgSO₄) und eingedampft. Der Rückstand wird über Kieselgel chromatographier (n-Hexan/Essigester 4:1). Man erhält die Verbindung (A17). 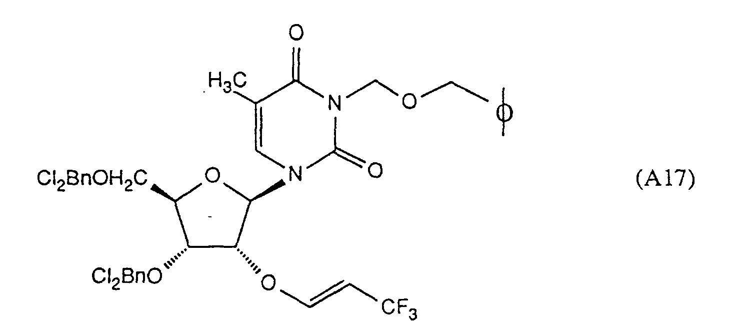 ¹H-NMR (250 MHz, CDCl₃): 7,72 [s, H-C(6)]; 6,80 (d, J=8Hz, CH=C); 5,82 [s, H-C(1')]; 5,48 (AB, CH₂); 4,85 (m, CH=C); 1,58 (s, CH₃).
MS:789 (M+H)⁺.

### Beispiel A18:

10,0 g der Verbindung (A17) werden in 200 ml THF gelöst und über 2 g Pd/C bei Raumtemperatur und Normaldruck hydriert. Nach 1 Stunde wird der Katalysator abfiltriert und das Filtrat eingedampft. Der Rückstand wird analog dem Beispiel A15 mit NaOMe nachbehandelt und chromatographiert (Kieselgel; n-Hexan/Essigsäureethylester 2:1). Man erhält die Verbindung (A18). 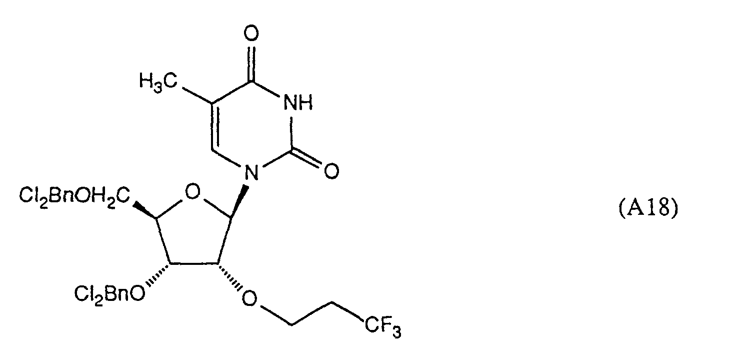 ¹H-NMR (CDCl₃): 9,00 (s, NH); 7,66 [s, H-C(6)]; 5,93 [d, J=1,5 Hz, H-C(1')]; 1,61 (s, CH3).
¹⁹F-NMR (CDCl₃): -65,23.
MS: 705 (M+Cl)⁻.

### Beispiel A19:

8,9 g der Verbindung (A6) werden in 90 ml THF gelöst und mit 0,34 g NaH (100 %) kurz aufgekocht. Nach dem Abklingen der H₂-Entwicklung werden bei 20°C 5,1 g 1,1,2-Trichlor-3,3,3-trifluorpropen zugetropft und anschliessend 5 Stunden bei 55°C gerührt. Das Reaktionsgemisch wird auf Wasser gegossen und mit Essigsäureethylester extrahiert. Nach dem Eindampfen wird chromatographiert (Kieselgel, Toluol/Essigsäureethylester 4:1). Man erhält die Verbindung (A19) als cis/trans(etwa 1:1)-Gemisch. 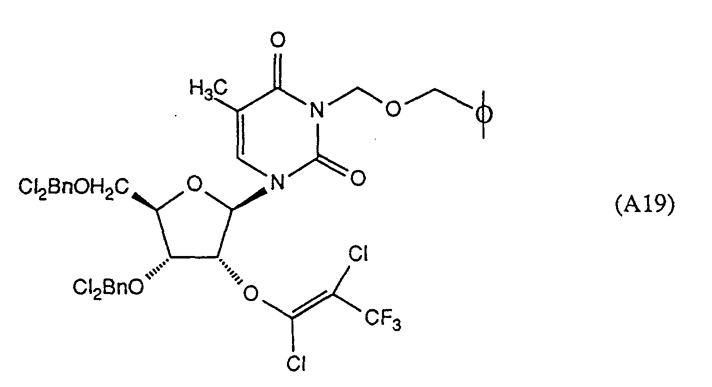 ¹H-NMR (CDCl₃): 7,63 und 7,60 [je s, je H-C(6)]; 5,91 und 5,87 [je s, je H-C(1')]; 1,61 und 1,57 (je s, je CH₃).
MS: 891 (M+Cl)⁻.

### Beispiel A20:

Wird die Verbindung (A18) oder (A19) analog den obigen Beispielen entschützt und in das Phosphoramidit überführt, so erhält man die Verbindung (A20) (Diastereoisomere 1:1). 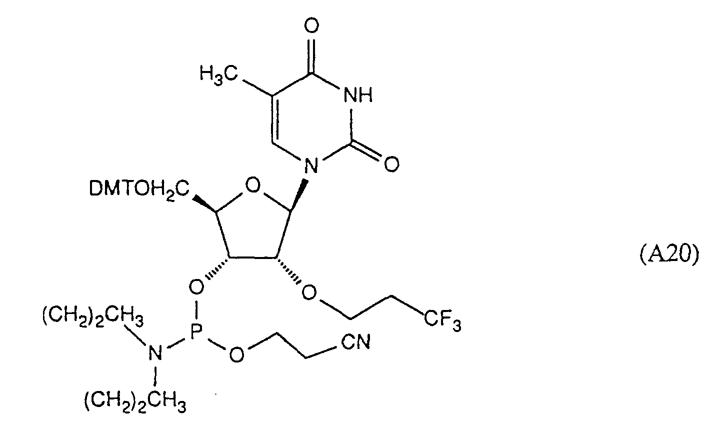 ¹H-NMR (CDCl₃): 8,6 (breites s, NH); 7,74 und 7,69 [je s, je H-C(6)]; 5,97 und 5,94 [je d, J=4 Hz, je H-C(1')].
³¹P-NMR (CDCl₃): 150,287 und 150,035 ppm.

### Beispiel B: Herstellung von Oligonukleotiden

Oligonukleotide werden unter Verwendung der erfindungsgemässen dimethoxytritylierten und 3'-aktivierten [3'-(β-Cyanoethoxy-di(i-propylamino)phosphoramidit)] Nukleoside bzw. solchen natürlichen aktivierten Nukleosiden an einen festen Träger gebunden (Controlled Pore Glas, CPG) und die Synthese auf einem DNA-Synthesiser (Applied Biosystems, Modell 380 B, Standard Phosphoramiditchemie und Iodoxidation) gemäss den Standardprotokollen des Herstellers durchgeführt [vergleiche auch "Oligonucleotide synthesis a practical approach" M.J Gait; IRL Press 1984 (Oxford-Washington DC)]. Nach der Kopplung des letzten Nukleosidbausteins wird das 5'-geschützte Oligonukleotid unter gleichzeitiger Abspaltung aller übrigen Schutzgruppen durch Behandlung mit konzentriertem wässrigem Ammoniak über Nacht vom Träger abgelöst und anschliessend unter Verwendung von 50 mM Ammoniumacetatpuffer (pH 7)/Acetonitril durch "reverse-phase" HPLC gereinigt. Anschliessend wird die 5'-Dimethoxytrityl-Schutzgruppe durch 20-minütige Behandlung mit 80 %-iger wässriger Essigsäure abgespalten, das Oligonukleotid mit Ethanol ausgefällt und durch Zentrifugation isoliert. Die Reinheit des Oligonukleotids wird durch Gelelektrophorese (Polyacrylamid), seine Identität mittels matrixunterstützter Laserdesorptions-Time-of-flight Massenspektroskopie (MALDI-TOF MS) überprüft.

### Beispiel C1: Affinität; Wechselwirkung der Oligonukleotide (Antisense) mit komplementären Oligoribonukleotidsequenzen (Sense)

Die Wechselwirkung der Oligonukleotide mit den entsprechenden basenkomplementären Oligomeren der natürlichen Ribonukleotide wird durch das Aufzeichnen von UV-Schmelzkurven und den daraus ermittelten Tₘ-Werten charakterisiert. Diese Standardmethode ist zum Beispiel von Marky, L.A., Breslauer, K.J., Biopolymers 26:1601-1620 (1987) beschrieben.
Es wird eine Lösung der Oligonukleotide und der entsprechenden basenkomplementären natürlichen Oligoribonukleotide in 10 mM Phosphatpuffer, 100 mM NaCl, 0,1 mM EDTA, pH = 7,0 (c = 4·10⁻⁶ M/Oligonukleotid) hergestellt und die Änderung der Extinktion bei 260 nm in Abhängigkeit von der Temperatur (15 bis 95°C) aufgezeichnet Aus den erhaltenen Schmelzkurven wird der Tₘ-Wert ermittelt (Tabelle 3).

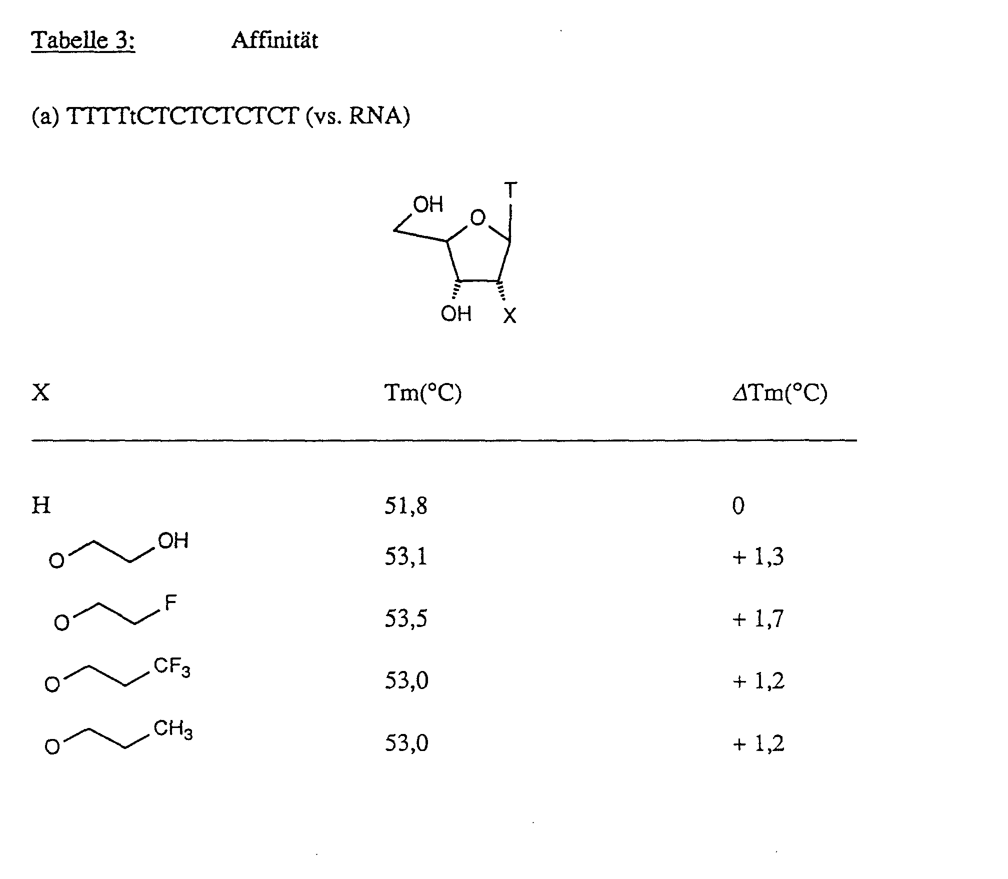

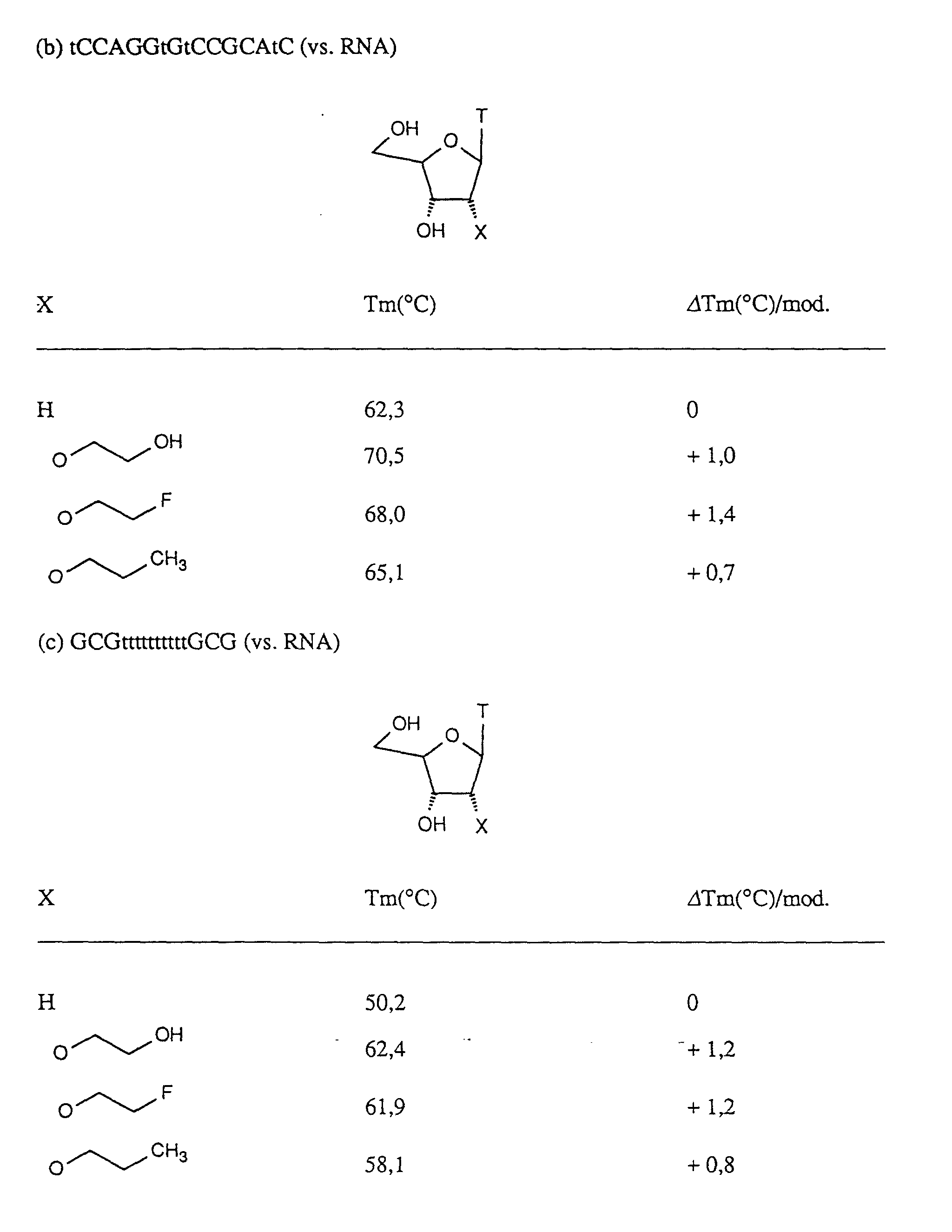

### Beispiel D2: Spezifität; Wechselwirkung des Oligonukleotids mit basenkomplementären Oligoribonukleotiden, in welche ein falsches Nukleosid (Y) eingebaut wurde

Man stellt Lösungen des Oligonukleotids mit den entsprechenden basenkomplementären Oligonukleotiden der Sequenzen r(GGA CCG GAA YGG TAC GAG) in 10 mM Phosphatpuffer, 100 mM NaCl, 0,1 mM EDTA, pH 7, (c = 4·10⁻⁶ M/Oligonukleotid) her und misst die Änderung der Extinktion bei 260 nm in Abhängigkeit von der Temperatur (15°C bis 95°C). Aus den Kurven wird der Tₘ-Wert ermittelt. Die Ergebnisse sind in der Tabelle 4 angegeben.

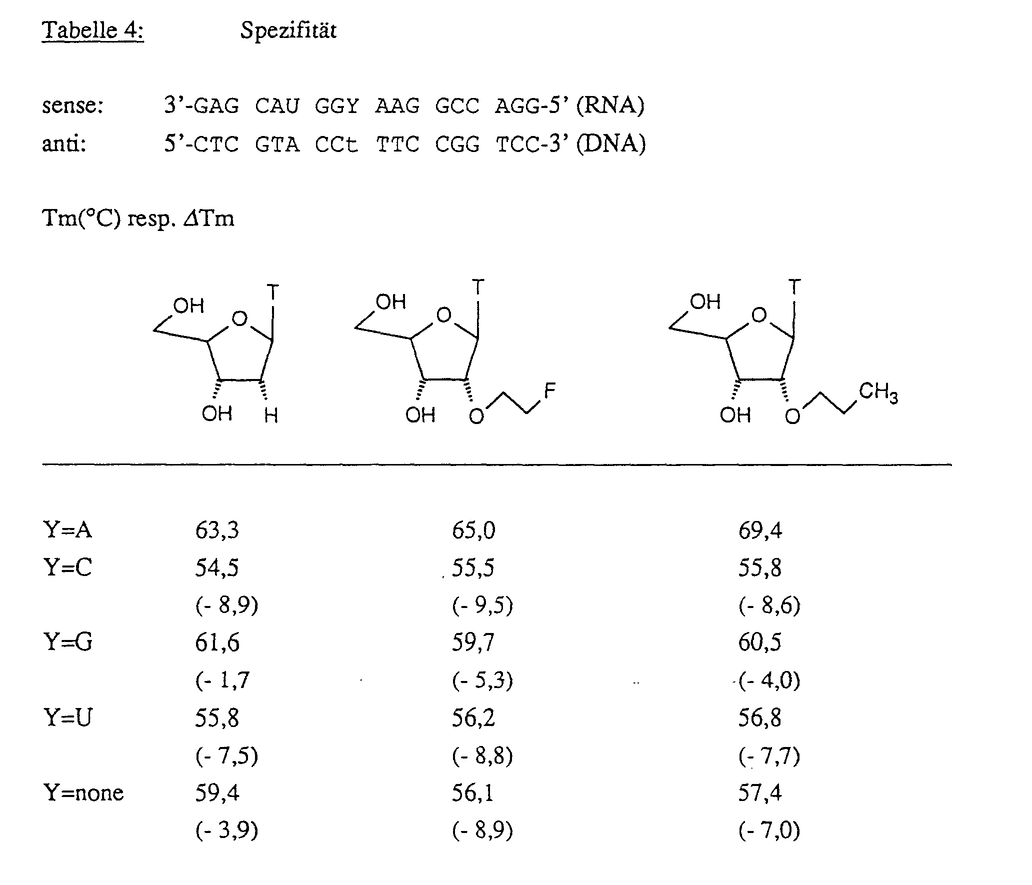

### Beispiel C3: Nucleasestabilität; Enzymatische Hydrolyse verschiedener Oligonukleotide der Sequenz d(TCC AGG TGT CCG ttt C)

Je 14 µg des synthetischen Oligonukleotids beziehungsweise des entsprechenden natürlichen Oligomers werden in 200 µl 10%-igem Hitze-inaktivierten Serum aus Kalbsföten bei 37°C inkubiert (c = 70 µg/ml). Nach 0,5; 1; 2; 4; 6, 24 und 48 Stunden werden jeweils 15 µl der Reaktionslösung durch Zugabe zu 25 µl 9 M Harnstoff und Trisborat-Puffer (pH 7) gequencht und bis zur Messung bei -20°C gelagert. Die gequenchten Reaktionslösungen werden mittels Polyacrylamid-Gelelektophorese aufgetrennt und die Spaltprodukte über den Phosphorgehalt bestimmt (Phospho-imagers-Methode). Das Verhältnis R der Summe der Konzentrationen des völlig intakten Oligonukleotids (cₙ^{(t)}) und des durch Abspaltung des natürlichen C-Bausteins vom 3'-Ende entstehenden Fragments (cₙ₋₁^{(t)}) zu einer gegebenen Zeit t zur Ausgangskonzentration des völlig intakten Oligonukleotids zum Zeitpunkt t = 0 (cₙ⁽⁰⁾) R = (cₙ^{(t)} + cₙ₋₁^{(t)})/cₙ⁽⁰⁾ wird gegen die Zeit graphisch aufgetragen. Die dabei ermittelten Halbwertszeiten τ_{1/2} - das sind jene Zeiten, für die R=0,5 - betragen

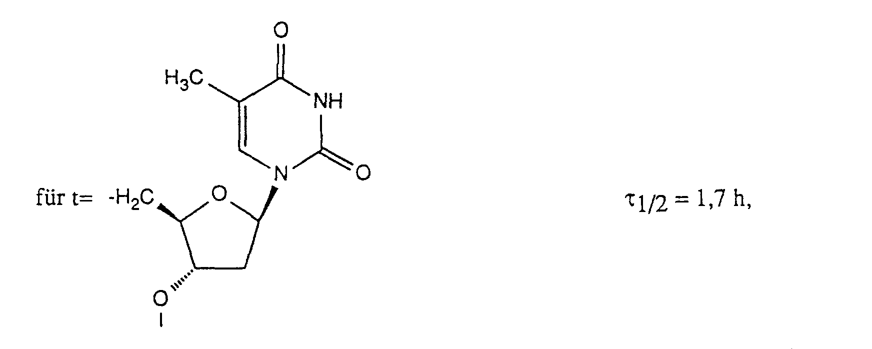

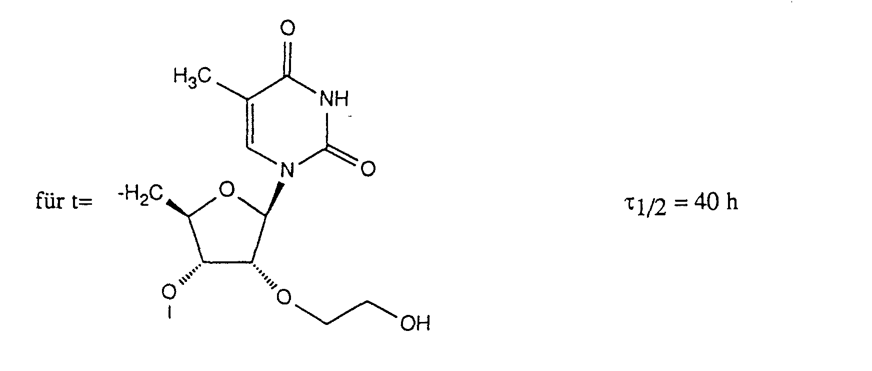

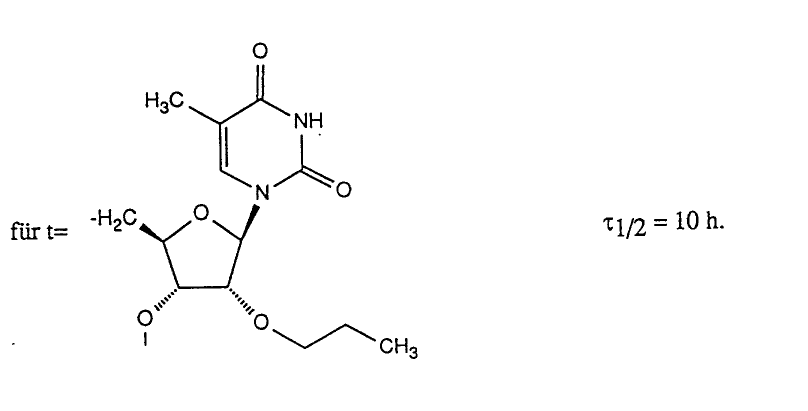

## Patentansprüche

1. Verbindungen der Formel I 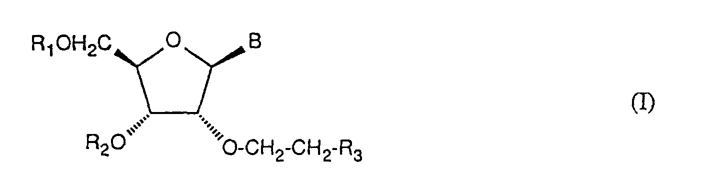 worin R₁ und R₂ unabhängig voneinander für Wasserstoff oder eine Schutzgruppe stehen oder R₁ diese Bedeutungen hat und R₂ für einen eine phosphorhaltige Nukleotid-Brückengruppe bildenden Rest steht; B einen Purin- oder Pyrimidinrest oder ein Analoges davon bedeutet; und R₃ für OH, F oder (CF₂)ₙCF₃ steht, worin n eine Zahl von 0 bis 7 bedeutet.

2. Verbindungen gemäss Anspruch 1, **dadurch gekennzeichnet, dass** R₁ und R₂ je für Wasserstoff stehen.

3. Verbindungen gemäss Anspruch 1, **dadurch gekennzeichnet, dass** R₁ und R₂ gleiche Schutzgruppen bedeuten.

4. Verbindungen gemäss Anspruch 1, worin B als Purinrest oder einem Analogen davon einen Rest der Formel II, IIa, IIb, IIc, IId, IIe oder IIf darstellt, 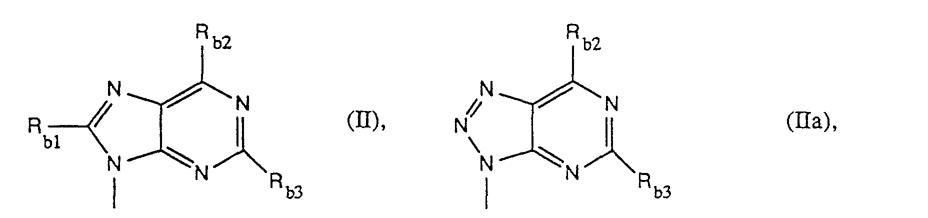 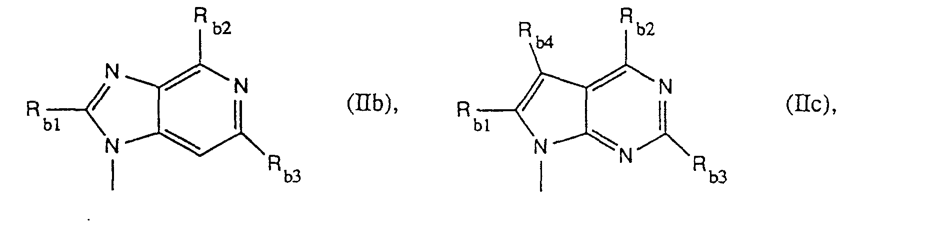 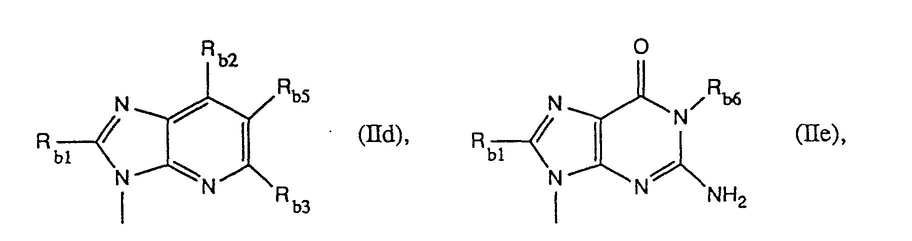 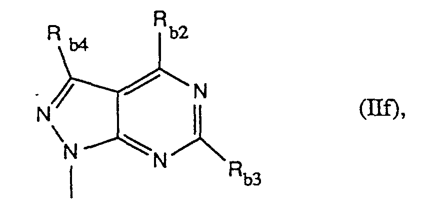 worin R_{b1} für H, Cl, Br, OH oder -O-C₁-C₁₂-Alkyl steht, und R_{b2}, R_{b3} und R_{b5} unabhängig voneinander H, OH, SH, NH₂, NHNH₂, NHOH, NHO-C₁-C₁₂-Alkyl,
-N=CH-N(C₁-C₁₂-Alkyl)₂, -N=CH-Azacycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, R_{b4} Wasserstoff, CN oder -C≡C-R_{b7} sowie R_{b6} und R_{b7} Wasserstoff oder C₁-C₄-Alkyl darstellen.

5. Verbindungen gemäss Anspruch 4, worin die Schutzgruppe für Hydroxyl- und Aminogruppen C₁-C₈-Acyl darstellt.

6. Verbindungen gemäss Anspruch 4, worin das Primäramino 1 bis 12 C-Atome und das Sekundäramino 2 bis 12 C-Atome enthält.

7. Verbindungen gemäss Anspruch 4, worin es sich bei dem Primäramino und Sekundäramino um Reste der Formel Rₐ₁Rₐ₂N handelt, worin Rₐ₁ für H steht oder unabhängig die Bedeutung von Rₐ₂ hat, und Rₐ₂ C₁-C₂₀-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-Alkenyl; Phenyl, Mono- oder Di-(C₁-C₄-Alkyl- oder Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder Rₐ₁ und Rₐ₂ zusammen Tetra- oder Pentamethylen, 3-Oxa-1,5-pentylen, -CH₂-NRₐ₃-CH₂CH₂oder -CH₂CH₂-NRₐ₃-CH₂CH₂- darstellen, worin Rₐ₃ für H oder C₁-C₄-Alkyl steht, wobei die Aminogruppe im Aminoalkyl unsubstituiert oder mit ein oder zwei C₁-C₄-Alkyl oder -Hydroxyalkylgruppen substituiert ist, und die Hydroxylgruppe im Hydroxyalkyl gegebenenfalls mit C₁-C₄-Alkyl verethert ist.

8. Verbindungen gemäss Anspruch 6, worin es sich bei dem Primäramino und Sekundäramino um Methyl-, Ethyl-, Dimethyl-, Diethyl-, Allyl-, Mono- oder Di-(hydroxyeth-2-yl)-. Phenyl- und Benzyl-, Acetyl-, Isobutyryl und Benzoylamino handelt.

9. Verbindungen gemäss Anspruch 4, worin R_{b1} in den Formeln II, IIb, IIc, IId und IIe für Wasserstoff steht

10. Verbindungen gemäss Anspruch 4, worin R_{b5} in Formel IId für Wasserstoff steht.

11. Verbindungen gemäss Anspruch 4, worin R_{b2} und R_{b3} in den Formeln II, IIa, IIb, IIc, IId und IIf unabhängig voneinander H, F, Cl, Br, OH, SH, NH₂, NHOH, NHNH₂, Methylamino, Dimethylamino, Benzoylamino, Methoxy, Ethoxy und Methylthio darstellen.

12. Verbindungen gemäss Anspruch 4, worin B ein Purinrest oder ein Rest eines Purinanalogen aus der Reihe Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin und N-Isobutyrylguanin ist.

13. Verbindungen gemäss Anspruch 1, worin B in Formel I als Pyrimidinrest einen Uracil-, Thymin- oder Cytosinrest der Formel III, IIIa, IIIb oder IIIc darstellt, 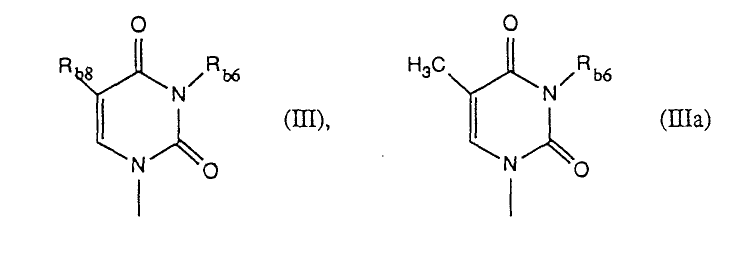 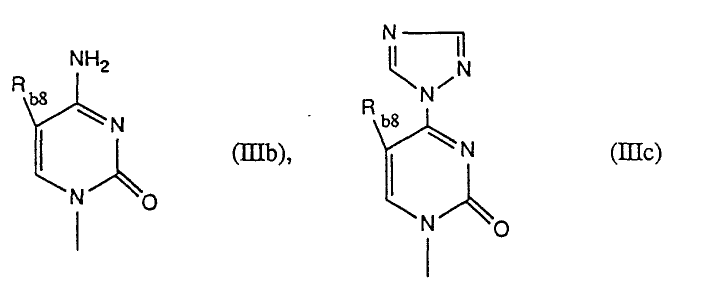 worin R_{b6} H oder C₁-C₄-Alkyl und R_{b8} H, OH, SH, NH₂, NHNH₂, NHOH, NHO-C₁-C₁₂-Alkyl, -N=CH-N(C₁-C₁₂-Alkyl)₂, -N=CH-Azacycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen, Sekundäramino mit 2 bis 30 C-Atomen, C₁-C₁₂-Alkenyl oder C₁-C₁₂-Alkinyl bedeuten, und die NH₂-Gruppe in Formel IIIb unsubstituiert oder mit C₁-C₆-Alkyl, Benzoyl oder einer Schutzgruppe substituiert ist, sowie die Dihydroderivate der Reste der Formeln III, IIIa, IIIb und IIIc.

14. Verbindungen gemäss Anspruch 13, worin R_{b8} H, C₁-C₆-Alkyl oder -Hydroxyalkyl, C₂-C₆-Alkenyl oder -Alkinyl, F, Cl, Br, NH₂, Benzoylamino, Mono- oder Di-C₁-C₆-alkylamino darstellt.

15. Verbindungen gemäss Anspruch 13, worin R_{b8} H, C₁-C₆-Alkyl oder -Alkoxy oder -Hydroxyalkyl, C₂-C₆ Alkenyl oder Alkinyl, F, Cl, Br, NH₂, Benzoylamino, Mono- oder Di-C₁-C₆-alkylamino darstellt.

16. Verbindungen gemäss Anspruch 14, worin R_{b8} H, F, Cl, Br, NH₂, NHCH₃, N(CH₃)₂, C₁-C₄-Alkyl, C₂-C₄-Alkenyl-(1) oder C₂-C₄-Alkinyl-(1) bedeutet.

17. Verbindungen gemäss Anspruch 15, worin R_{b8} H, C₁-C₄-Alkyl, C₂-C₄-Alkenyl-(1), C₂-C₄-Alkinyl-(1), NH₂, NHCH₃ oder (CH₃)₂N bedeutet.

18. Verbindungen gemäss Anspruch 1, worin B als Rest eines Pyrimidinanalogen sich von Uracil, Thymin, Cytosin, 5-Fluoruracil, 5-Chloruracil, 5-Bromuracil, Dihydrouracil, 5-Methylcytosin, 5-Propinthymin und 5-Propincytosin ableitet.

19. Verbindungen gemäss Anspruch 1, **dadurch gekennzeichnet, dass** R₂ als phosphorhaltiger eine Nukleotid-Brückengruppe bildender Rest der Formel P1 oder P2 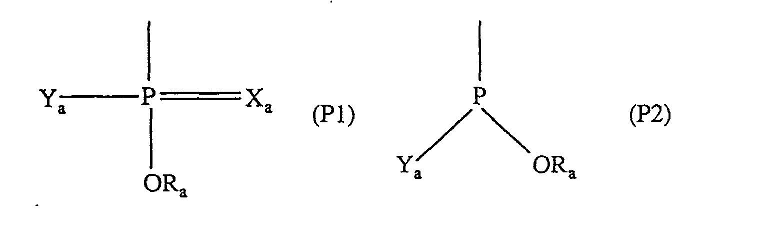 entspricht, worin Yₐ Wasserstoff, C₁-C₁₂-Alkyl, C₆-C₁₂-Aryl, C₇-C₂₀-Aralkyl, C₇-C₂₀-Alkaryl, -ORb, -SR_{b}, -NH₂, Primäramino, Sekundäramino, O^{⊖}M^{⊕} oder S^{⊖}M^{⊕} darstellt; Xₐ Sauerstoff oder Schwefel bedeutet; Rₐ Wasserstoff, M^{⊕}, C₁-C₁₂-Alkyl, C₂-C₁₂-Alkenyl, C₆-C₁₂-Aryl, oder die Gruppe RₐO- für N-Heteroaryl-N-yl mit 5 Ringgliedern und 1 bis 3 N-Atomen steht; Rb Wasserstoff, C₁-C₁₂-Alkyl oder C₆-C₁₂-Aryl bedeutet; und M^{⊕} für Na^{⊕}, K^{⊕}, Li^{⊕}, NH₄^{⊕} steht oder Primär-, Sekundär-, Tertiär- oder Quatenärammonium darstellt; wobei Alkyl, Aryl, Aralkyl und Alkaryl in Yₐ, Rₐ und R_{b} unsubstituiert oder mit Alkoxy, Alkylthio, Halogen, -CN, -NO₂, Phenyl, Nitrophenyl, oder Halogenphenyl substituiert ist.

20. Verbindungen gemäss Anspruch 19, **dadurch gekennzeichnet, dass** Rₐ β-Cyanoethyl bedeutet, und Yₐ Di(i-propyl)amino darstellt.

21. Verbindung gemäss Anspruch 1, **dadurch gekennzeichnet, dass** R₃ OH bedeutet.

22. Verbindungen gemäss Anspruch 1, **dadurch gekennzeichnet, dass** R₃ F bedeutet.

23. Verbindungen gemäss Anspruch 1, **dadurch gekennzeichnet, dass** R₃ (CF₂)ₙCF₃ und n eine Zahl von 0 bis 7 bedeutet.

24. Verbindungen gemäss Anspruch 23, **dadurch gekennzeichnet, dass** n die Zahl 0 bedeutet.

25. Verfahren zur Herstellung von Verbindungen der Formel I, 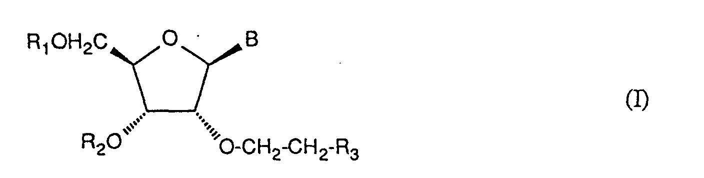 worin R₁ und R₂ unabhängig voneinander für Wasserstoff oder eine Schutzgruppe stehen; B einen Purin- oder Pyrimidinrest oder ein Analoges davon bedeutet; und
(a) R₃ OH bedeutet,
**dadurch gekennzeichnet, dass** man eine Verbindung der Formel IVa 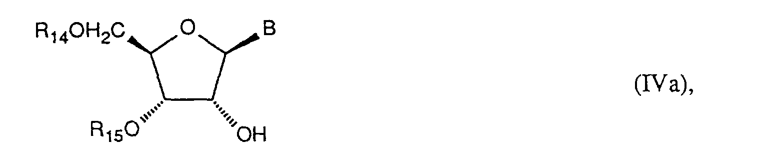 worin R₁₄ und R₁₅ für gleiche oder verschiedene Schutzgruppen stehen und B einen Purinoder Pyrimidinrest oder ein Analoges davon bedeutet, wobei funktionelle Gruppen im Basenrest B durch Schutzgruppen geschützt sind, in einem inerten Lösungsmittel mit einer Verbindung der Formel A umsetzt
X-CH₂-COOR₄ (A),
worin R₄ C₁-C₄-Alkyl und X Cl, Br, I, Tosyl-O oder Mesyl-O bedeutet;
und die Esterfunktion anschliessend mit NaBH₄ oder LiAlH₄ reduziert, wobei die entstehende OH-Gruppe vorübergehend mit einer Gruppe wie für R₁ definiert geschützt werden kann;
(b) R₃ F bedeutet,
**dadurch gekennzeichnet, dass** man eine Verbindung der Formel I, worin R₃ OH bedeutet, mit einer Verbindung der Formel B umsetzt 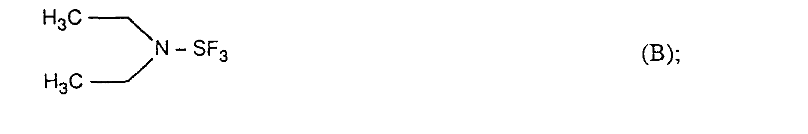
(c) R₃ -(CF₂)ₙ-CF₃ bedeutet, worin n für eine Zahl von 0 bis 7 steht,
**dadurch gekennzeichnet, dass** man eine Verbindung der Formel IVa mit einer Verbindung der Formel C, D oder E umsetzt
CH≡C-(CF₂)ₙ-CF₃ (C)
CH₂=CH-(CF₂)ₙ-CF₃ (D) 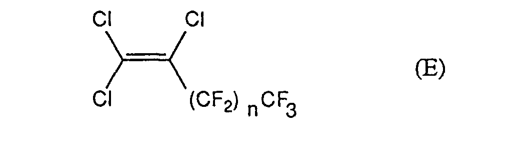
und anschliessend die gegebenenfalls vorhandene Doppelbindung oder chlorierte Doppelbindung katalytisch zu CH₂CH₂(CF)ₙCF₃ reduziert;
(d) R₃ OH, F oder -(CF₂)ₙ-CF₃ bedeutet, worin n für eine Zahl von 0 bis 7 steht,
**dadurch gekennzeichnet, dass** man eine Verbindung der Formel IVb 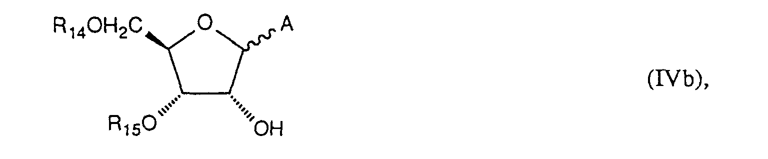 worin R₁₄ und R₁₅ die oben erwähnte Bedeutung haben und A für eine Abgangsgruppe steht, nach einem der in (a) bis (c) beschriebenen Verfahren an der 2'-OH Gruppe substituiert und den Basenrest B durch Substitution einführt; und gegebenenfalls die Schutzgruppen R₁₄ und R₁₅ abspaltet.

26. Verwendung der Verbindungen gemäss Anspruch 1 zur Herstellung von Oligonukleotiden, die gleiche oder verschiedene Monomereinheiten von Verbindungen der Formeln I oder die mindestens eine Monomereinheit von Verbindungen der Formeln I in Kombination mit Monomereinheiten von anderen natürlichen oder synthetischen Nukleosiden enthalten, wobei die Oligonukleotide aus 2 bis 200 Monomereinheiten bestehen.

27. Verwendung gemäss Anspruch 26 zur Herstellung von Oligonukleotiden mit 2 bis 100 Monomereinheiten.

28. Verwendung gemäss Anspruch 27 zur Herstellung von Oligonukleotiden mit 2 bis 50 Monomereinheiten.

29. Verwendung gemäss Anspruch 28 zur Herstellung von Oligonukleotiden mit 4 bis 30 Monomereinheiten.

30. Verwendung gemäss Anspruch 26 zur Herstellung von Oligonukleotiden mit gleichen oder verschiedenen Monomereinheiten von Verbindungen der Formeln I.

31. Verwendung gemäss Anspruch 26 zur Herstellung von Oligonukleotiden mit gleichen Monomereinheiten von Verbindungen der Formeln I und Monomereinheiten von natürlichen oder synthetischen Nukleosiden.

32. Oligonukleotide der Formel V
5'-U-(O-Y-O-V-)ₓO-Y-O-W-3' (V),
worin x eine Zahl von 0 bis 200 und Y eine Nukleotid-Brückengruppe bedeuten; U, V und W je für sich gleiche oder verschiedene Reste von natürlichen oder synthetischen Nukleosiden darstellen und mindestens einer der Reste U, V und/oder W einen Rest der Formel VI bedeutet 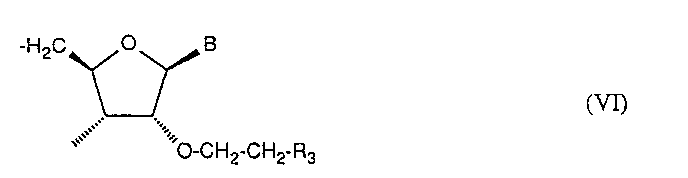 worin B einen Purin- oder Pyrimidinrest oder ein Analoges davon darstellt; und R₃ für OH, F oder (CF₂)ₙCF₃ steht, worin n eine Zahl von 0 bis 7 bedeutet.

33. Oligonukleotide gemäss Anspruch 32, **dadurch gekennzeichnet, dass** n die Zahl 0 bedeutet.

34. Oligonukleotide gemäss Anspruch 32, **dadurch gekennzeichnet, dass** es sich bei der Brückengruppe Y um -P(O)O^{⊖}-, -P(O)S^{⊖}-, -P(S)S^{⊖}-, -P(O)R₁₆-, -P(O)NR₁₇R₁₈-, oder -CH₂- handelt, worin R₁₆ H oder C₁-C₆-Alkyl darstellt, und R₁₇ und R₁₈ unabhängig voneinander die Bedeutung von R₁₆ haben.

35. Oligonukleotide gemäss Anspruch 32, **dadurch gekennzeichnet, dass** sich bei der Brückengruppe Y um -P(O)O^{⊖}- handelt.

36. Oligonukleotide gemäss Anspruch 32, **dadurch gekennzeichnet, dass** x für eine Zahl von 0 bis 100 steht.

37. Oligonukleotide gemäss Anspruch 36, **dadurch gekennzeichnet, dass** x für eine Zahl von 1 bis 50 steht.

38. Oligonukleotide gemäss Anspruch 37, **dadurch gekennzeichnet, dass** x für eine Zahl von 3 bis 29 steht.

39. Oligonukleotide gemäss Anspruch 32, **dadurch gekennzeichnet, dass** die Reste der Formel VI endständig und/oder in der Nukleotidsequenz gebunden sind.

40. Oligonukleotide gemäss Anspruch 32, **dadurch gekennzeichnet, dass** die Reste der Formel VI zwischen Resten von natürlichen oder synthetischen Nukleosiden gebunden sind.

41. Oligonukleotide gemäss den Ansprüchen 39 und 40, **dadurch gekennzeichnet, dass** 2 bis 5 gleiche oder verschiedene Reste der Formel VI aufeinander folgen.

42. Oligonukleotide gemäss Anspruch 32, **dadurch gekennzeichnet, dass** insgesamt 4 bis 30 Nukleosideinheiten und 1 bis 12 Reste der Formel VI enthalten sind.

43. Oligonukleotide gemäss Anspruch 32, worin B als Purinrest oder einem Analogen davon einen Rest der Formeln II, IIa, IIb, IIc, IId, IIe oder IIf darstellt, 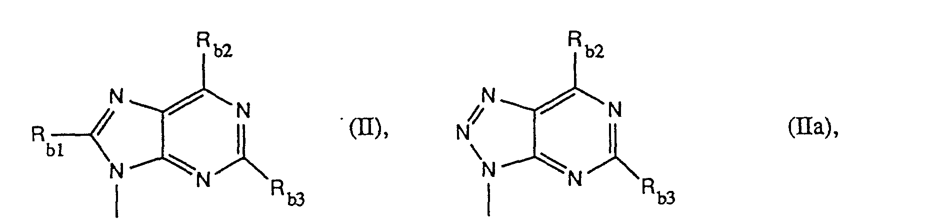 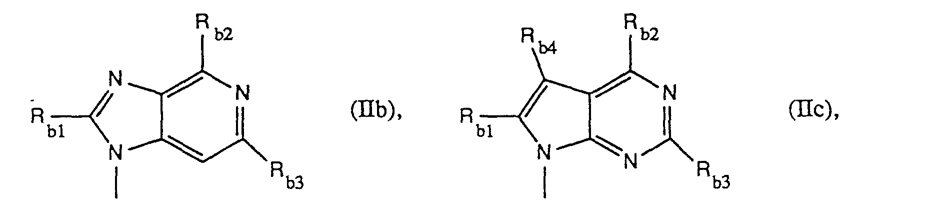 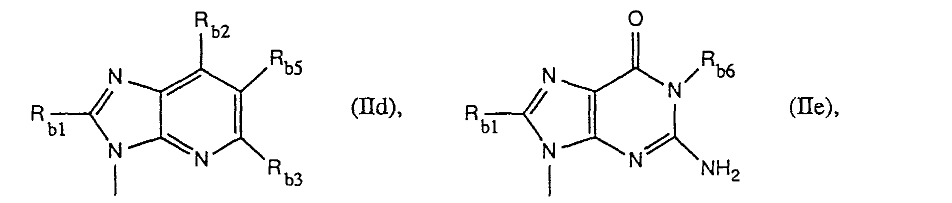 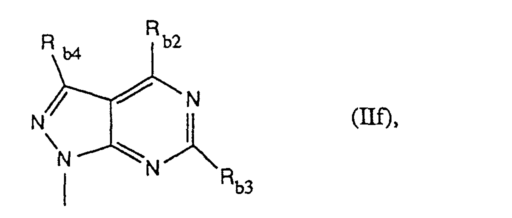 worin R_{b1} für H, Cl, Br, OH oder -O-C₁-C₁₂-Alkyl steht, und R_{b2}, R_{b3} und R_{b5} unabhängig voneinander H, OH, SH, NH₂, NHNH₂, NHOH, NHO-C₁-C₁₂-Alkyl,
-N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, R_{b4} Wasserstoff, CN oder -C≡C-R_{b7}, R_{b6} und R_{b7} Wasserstoff oder C₁-C₄-Alkyl darstellen.

44. Oligonukleotide gemäss Anspruch 32, worin B einen Uracil-, Thymin- oder Cytosinrest der Formel III, IIIa, IIIb oder IIIc darstellt, 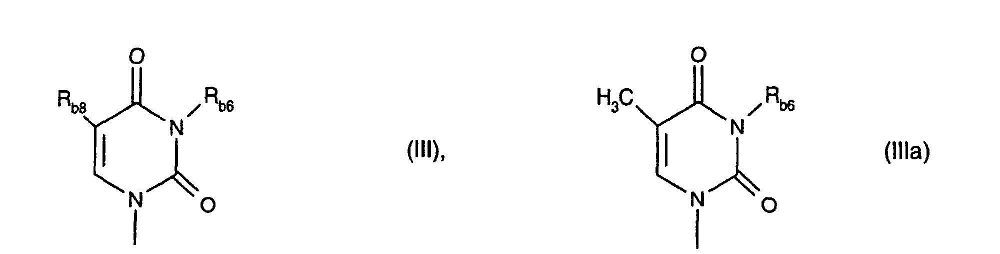 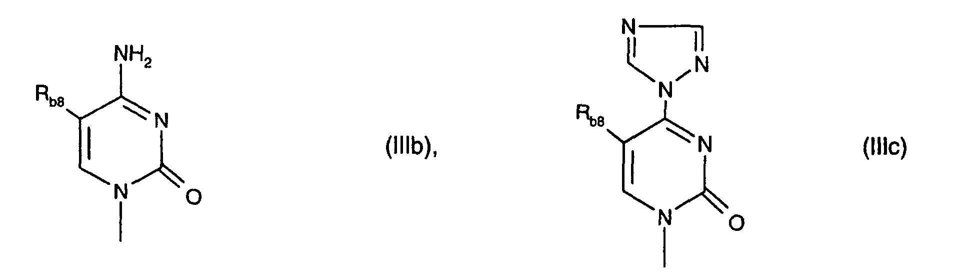 worin R_{b6} H oder C₁-C₄-Alkyl und R_{b8} H, OH, SH, NH₂, NHNH₂, NHOH, NHO-C₁-C₁₂-Alkyl, - N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen, Sekundäramino mit 2 bis 30 C-Atomen, C₁-C₁₂-Alkenyl oder C₁-C₁₂-Alkinyl bedeuten, und die NH₂-Gruppe in Formel IIIb unsubstituiert oder mit C₁-C₁-C₆-Alkyl, Benzoyl oder einer Schutzgruppe substituiert ist, sowie die Dihydroderivate der Reste der Formeln III, IIIa, IIIb und IIIc.

45. Oligonukleotide gemäss Anspruch 32, worin R₃ OH bedeutet.

46. Oligonukleotide gemäss Anspruch 32, worin R₃ F bedeutet.

47. Oligonukleotide gemäss Anspruch 32, worin R₃ (CF₂)ₙCF₃ bedeutet.

48. Oligonukleotide gemäss Anspruch 32, worin n eine Zahl von 0 bis 3 bedeutet.

49. Oligonukleotide gemäss Anspruch 48, worin n 0 bedeutet.

50. Verwendung der Oligonukleotide der Formel V als Diagnostika zum in vitro Nachweis von viralen Infektionen oder genetisch bedingten Krankheiten.

51. Pharmazeutisches Präparat, enthaltend eine wirksame Menge eines Nukleosids der Formeln I oder eines Oligonukleotids der Formel V alleine oder zusammen mit anderen Wirkstoffen, ein pharmazeutisches Trägermaterial und gegebenenfalls Hilfsstoffe.

## Claims

1. A compound of formula I 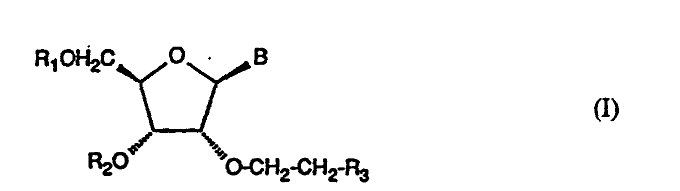 wherein R₁ and R₂ are each independently ofthe other hydrogen or a protecting group, or R₁ has those definitions and R₂ is a radical forming a phosphorus-containing nucleotide bridge group; B is a purine or pyrimidine radical or an analogue thereof; and R₃ is OH, F or (CF₂)ₙCF₃ wherein n is a number from 0 to 7.

2. A compound according to claim 1, wherein R₁ and R₂ are each hydrogen.

3. A compound according to claim 1, wherein R₁ and R₂ are identical protecting groups.

4. A compound according to claim 1, wherein B as a purine radical or an analogue thereof is a radical of formula II, IIa, IIb, IIc, IId, IIe or IIf 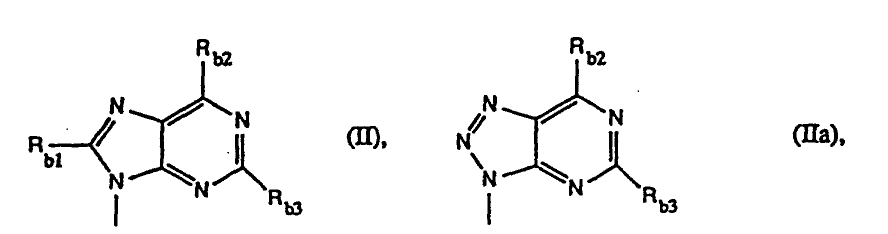 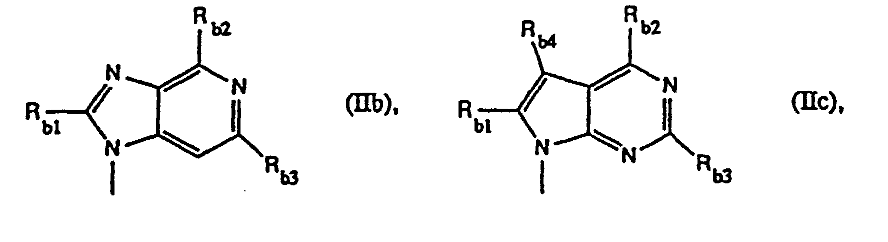 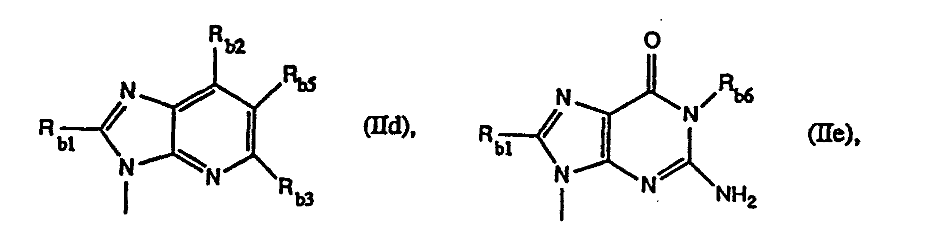 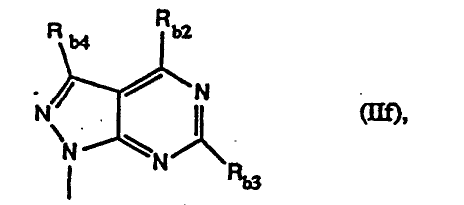 wherein R_{b1} is H, Cl, Br, OH or -O-C₁-C₁₂alkyl, and R_{b2}, R_{b3} and R_{b5} are each independently of the others H, OH, SH, NH₂, NHNH₂, NHOH, NHO-C₁-C₁₂alkyl, -N=CH-N-(C₁-C₁₂alkyl)₂, -N=CH-azacycloalkyl, F, Cl, Br, C₁-C₁₂alkyl, hydroxy-C₁-C₁₂alkyl, amino-C₁-C₁₂alkyl, C₁-C₁₂alkoxy, benzyloxy or C₁-C₁₂alkylthio, the hydroxy and amino groups being unsubstituted or substituted by a protecting group, phenyl, benzyl, primary amino having from 1 to 20 carbon atoms or secondary amino having from 2 to 30 carbon atoms, R_{b4} is hydrogen, CN or -C≡C-R_{b7}, and R_{b6} and R_{b7} are hydrogen or C₁-C₄alkyl.

5. A compound according to claim 4, wherein the protecting group for hydroxy and amino groups is C₁-C₈acyl.

6. A compound according to claim 4, wherein the primary amino contains from 1 to 12 carbon atoms and the secondary amino contains from 2 to 12 carbon atoms.

7. A compound according to claim 4, wherein the primary amino and secondary amino are radicals ofthe formula Rₐ₁Rₐ₂N wherein Rₐ₁ is H or, independently, has the definition of Rₐ₂, and Rₐ₂ is C₁-C₂₀-alkyl, -aminoalkyl or -hydroxyalkyl; carboxyalkyl or carbalkoxyalkyl wherein the carbalkoxy group contains from 2 to 8 carbon atoms and the alkyl group from 1 to 6, preferably from I to 4, carbon atoms; C₂-C₂₀alkenyl; phenyL mono- or di-(C₁-C₄-alkyl- or -alkoxy)phenyl, benzyl, mono- or di-(C₁-C₄-alkyl- or -alkoxy)benzyl; or 1,2-, 1,3- or 1,4-imidazolyl-C₁-C₆alkyl, or Rₐ₁ and Rₐ₂ together are tetra- or penta-methylene, 3-oxa-1,5-pentylene, -CH₂-NRₐ₃-CH₂CH₂- or -CH₂CH₂-NRₐ₃-CH₂CH₂- wherein Rₐ₃ is H or C₁-C₄alkyl, the amino group in aminoalkyl being unsubstituted or substituted by one or two C₁-C₄-alkyl or -hydroxyalkyl groups. and the hydroxy group in hydroxyalkyl being free or etherified by C₁-C₄alkyl.

8. A compound according to claim 6, wherein the primary amino and secondary amino are methyl-, ethyl-, dimethyl-, diethyl-, allyl-, mono- or di-(hydroxy-eth-2-yl)-, phenyl- and benzyl-, acetyl-, isobutyryl- or benzoyl-amino.

9. A compound according to claim 4, wherein R_{b1} in the formulae II, IIb, IIc, IId and IIe is hydrogen.

10. A compound according to claim 4, wherein R_{b5} in formula IId is hydrogen.

11. A compound according to claim 4, wherein R_{b2} and R_{b3} in the formulae II, IIa, IIb, IIc, IId and IIf are each independently of the other H, F, Cl, Br, OH, SH, NH₂, NHOH, NHNH₂, methylamino, dimethylamino, benzoylamino, methoxy, ethoxy or methylthio.

12. A compound according to claim 4, wherein B is a purine radical or a radical of a purine analogue from the series adenine, N-methyladenine, N-benzoyladenine, 2-methylthioadenine, 2-aminoadenine, 6-hydroxypurine, 2-amino-6-chloropurine, 2-amino-6-methylthiopurine, guanine and N-isobutyrylguanine.

13. A compound according to claim 1, wherein B in formula I as a pyrimidine radical is a uracil, thymine or cytosine radical of formula III, IIIa, IIIb or IIIc 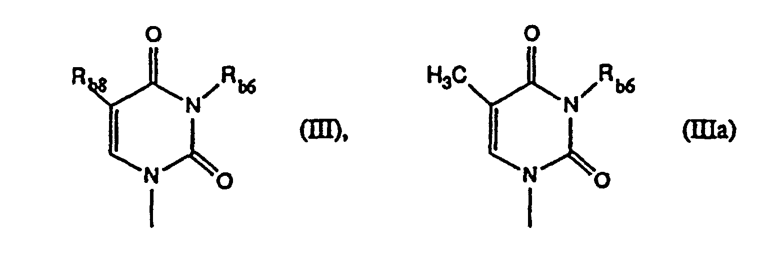 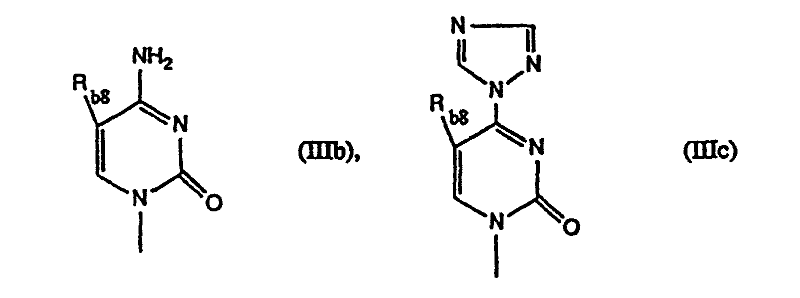 wherein R_{b6} is H or C₁-C₄alkyl and R_{b8} is H, OH, SH, NH₂, NHNH₂, NHOH, NHO-C₁-C₁₂alkyl, -N=CH-N(C₁-C₁₂alkyl)₂, -N=CH-azacycloalkyl, F, Cl, Br, C₁-C₁₂alkyl, hydroxy-C₁-C₁₂alkyl, amino-C₁-C₁₂alkyl, C₁-C₁₂alkoxy, benzyloxy or C₁-C₁₂alkylthio, the hydroxy and amino groups being unsubstituted or substituted by a protecting group, phenyl, benzyl, primary amino having from 1 to 20 carbon atoms, secondary amino having from 2 to 30 carbon atoms, C₁-C₁₂alkenyl or C₁-C₁₂alkynyl, and the NH₂ group in formula IIIb is unsubstituted or is substituted by C₁-C₆alkyl, benzoyl or by a protecting group, or a dihydro derivative of a radical of formula III, IIIa, IIIb or IIIc.

14. A compound according to claim 13, wherein R_{b8} is H, C₁-C₆-alkyl or -hydroxyalkyl, C₂-C₆-alkenyl or -alkynyl, F, Cl, Br, NH₂, benzoylamino, or mono- or di-C₁-C₆alkylamino.

15. A compound according to claim 13, wherein R_{b8} is H, C₁-C₆-alkyl or -alkoxy or -hydroxyalkyl, C₂-C₆-alkenyl or -alkynyl, F, Cl, Br, NH₂, benzoylamino, or mono- or di-C₁-C₆alkylamino.

16. A compound according to claim 14, wherein R_{b8} is H, F, Cl, Br, NH₂, NHCH₃, N(CH₃)₂, C₁-C₄alkyl, C₂-C₄alkenyl-(1) or C₂-C₄alkynyl-(1).

17. A compound according to claim 15, wherein R_{b8} is H, C₁-C₄alkyl, C₂-C₄alkenyl-(1), C₂-C₄alkynyl-(1), NH₂, NHCH₃ or (CH₃)₂N.

18. A compound according to claim 1, wherein B as the radical of a pyrimidine analogue is derived from uraciL thymine, cytosine, 5-fluorouracil, 5-chlorouracil, 5-bromouracil, dihydrouracil, 5-methylcytosine, 5-propynethymine and 5-propynecytosine.

19. A compound according to claim 1, wherein R₂ as a phosphorus-containing radical forming a nucleotide bridge group has the formula P1 or P2 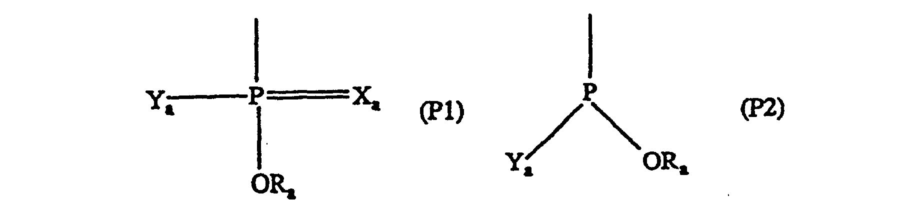 wherein Yₐ is hydrogen, C₁-C₁₂alkyl, C₆-C₁₂aryl, C₇-C₂₀aralkyl, C₇-C₂₀alkaryl, -OR_{b}, -SR_{b}, -NH₂, primary amino, secondary amino, O^{⊖}M^{⊕} or S^{⊖}M^{⊕}; Xₐ is oxygen or sulfur; Rₐ is hydrogen, M^{⊕}, C₁-C₁₂alkyl, C₂-C₁₂alkenyl, C₆-C₁₂aryl, or the group RₐO- is N-heteroaryl-N-yl having 5 ring members and from 1 to 3 N atoms; R_{b} is hydrogen, C₁-C₁₂alkyl or C₆-C₁₂aryl; and M^{⊕} is Na^{⊕}, K^{⊕}, Li^{⊕}, NH₄^{⊕} or primary, secondary, tertiary or quaternary ammonium; alkyl, aryl, aralkyl and alkaryl in Yₐ, Rₐ and R_{b} being unsubstituted or substituted by alkoxy, alkylthio, halogen, -CN, -NO₂, phenyl, nitrophenyl or by halophenyl.

20. A compound according to claim 19, wherein Rₐ is β-cyanoethyl, and Yₐ is di(isopropyl)amino.

21. A compound according to claim 1, wherein R₃ is OH.

22. A compound according to claim 1, wherein R₃ is F.

23. A compound according to claim 1, wherein R₃ is (CF₂)ₙCF₃ and n is a number from 0 to 7.

24. A compound according to claim 23, wherein n is 0.

25. A process for the preparation of a compound of formula I 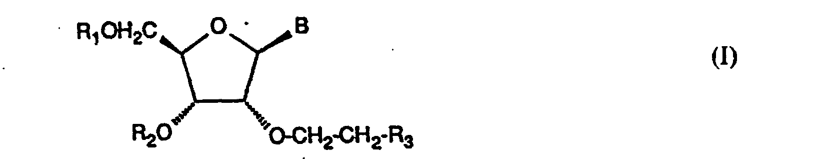 wherein R₁ and R₂ are each independently of the other hydrogen or a protecting group; B is a purine or pyrimidine radical or an analogue thereof; and
(a) R₃ is OH,
which comprises reacting a compound of formula IVa 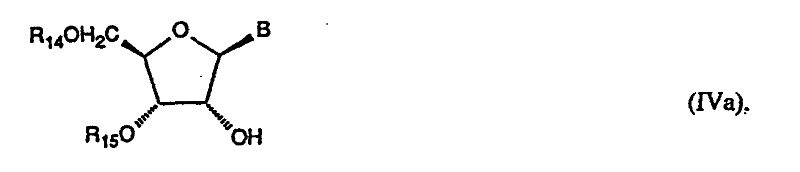 wherein R₁₄ and R₁₅ are identical or different protecting groups and B is a purine or pyrimidine radical or an analogue thereof and with functional groups in the base radical B being protected by protecting groups, in an inert solvent with a compound of formula A
X-CH₂-COOR₄ (A),
wherein R₄ is C₁-C₄alkyl and X is CL Br, I, tosyl-O or mesyl-O;
and subsequently reducing the ester function with NaBH₄ or LiAlH₄, it being possible to protect the resulting OH group temporarily with a group defined for R₁;
(b) R₃ is F,
which comprises reacting a compound of formula I wherein R₃ is OH with a compound of formula B 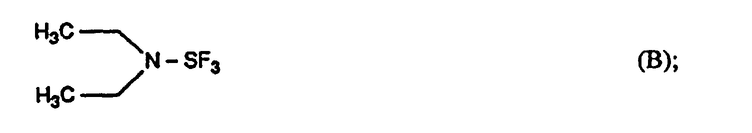
(c) R₃ is -(CF₂)ₙ-CF₃ wherein n is a number from 0 to 7.
which comprises reacting a compound of formula IVa with a compound of formula C, D or E
CH≡C-(CF₂)ₙ-CF₃ (C)
CH₂=CH-(CF₂)ₙ-CF₃ (D) 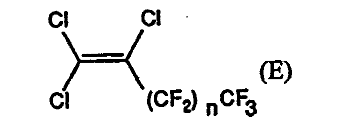
and subsequently catalytically reducing the double bond or chlorinated double bond that may be present to CH₂CH₂(CF)ₙCF₃;
(d) R₃ is OH, F or -(CF₂)ₙ-CF₃ wherein n is a number from 0 to 7.
which comprises substituting a compound of formula IVb 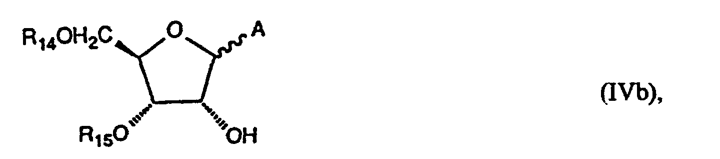
wherein R₁₄ and R₁₅ are as defined above and A is a leaving group. at the 2'-OH group by one of the methods described in (a) to (c), and introducing the base radical B by substitution; and, if desired, removing the protecting groups R₁₄ and R₁₅.

26. The use of a compound according to claim 1 for the preparation of an oligonucleotide that comprises identical or different monomer units of a compound of formula I or that comprises at least one monomer unit of a compound of formula I in combination with monomer units of other, natural or synthetic, nucleosides, the oligonucleotide consisting of from 2 to 200 monomer units.

27. The use according to claim 26 for the preparation of an oligonucleotide having from 2 to 100 monomer units.

28. The use according to claim 27 for the preparation of an oligonucleotide having from 2 to 50 monomer units.

29. Use according to claim 28 for the preparation of an oligonucleotide having from 4 to 30 monomer units.

30. Use according to claim 26 for the preparation of an oligonucleotide having identical or different monomer units of a compound of formula 1.

31. Use according to claim 26 for the preparation of an oligonucleotide having identical monomer units of a compound of formula I and monomer units of one or more natural or synthetic nucleosides.

32. An oligonucleotide of formula V
5'-U-(O-Y-O-V-)ₓO-Y-O-W-3' (V),
wherein x is a number from 0 to 200 and Y is a nucleotide bridge group; U, V and W are each independently identical or different radicals of natural or synthetic nucleosides and at least one ofthe radicals U, V and/or W is a radical of formula VI 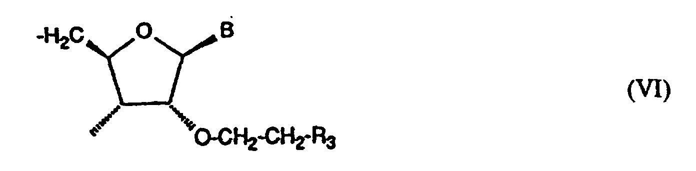 wherein B is a purine or pyrimidine radical or an analogue thereof and R₃ is OH, F or (CF₂)ₙCF₃ wherein n is a number from 0 to 7.

33. An oligonucleotide according to claim 32, wherein n is 0.

34. An oligonucleotide according to claim 32, wherein the bridge group Y is -P(O)O^{⊖}-, -P(O)S^{⊖}-, -P(S)S^{⊖}-, -P(O)R₁₆-, -P(O)NR₁₇R₁₈- or -CH₂- wherein R₁₆ is H or C₁-C₆alkyl and R₁₇ and R₁₈ each independently of the other have the definition of R₁₆.

35. An oligonucleotide according to claim 32, wherein the bridge group Y is -P(O)O^{⊖}-.

36. An oligonucleotide according to claim 32, wherein x is a number from 0 to 100.

37. An oligonucleotide according to claim 36, wherein x is a number from 1 to 50.

38. An oligonucleotide according to claim 37, wherein x is a number from 3 to 29.

39. An oligonucleotide according to claim 32, wherein the radicals of formula VI are bonded terminally and/or in the nucleotide sequence.

40. An oligonucleotide according to claim 32, wherein the radicals of formula VI are bonded between radicals of natural or synthetic nucleosides.

41. An oligonucleotide according to claims 39 and 40, wherein from 2 to 5 identical or different radicals of formula VI follow one another.

42. An oligonucleotide according to claim 32, wherein a total of from 4 to 30 nucleoside units and from 1 to 12 radicals of formula VI is present.

43. An oligonucleotide according to claim 32, wherein B as a purine radical or an analogue thereof is a radical of formula II, IIa, IIb, IIc, IId, IIe or IIf 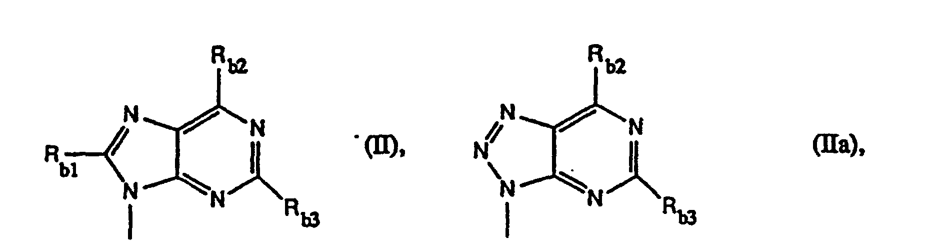 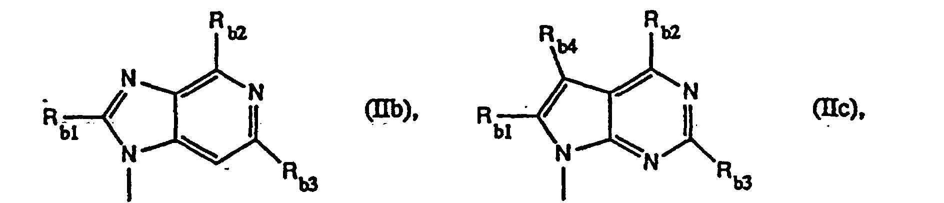 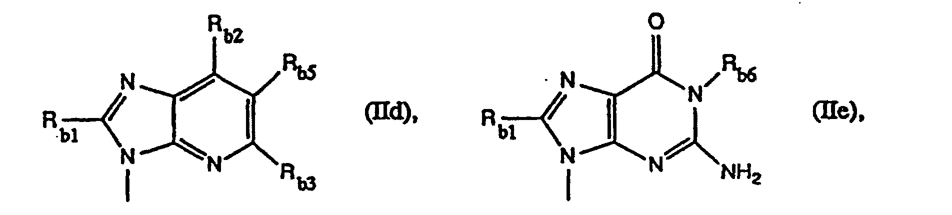 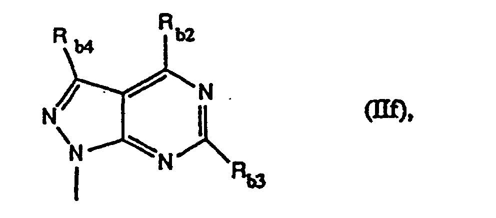 wherein R_{b1} is H, CL Br, OH or -O-C₁-C₁₂alkyl, and R_{b2}, R_{b3} and R_{b5} are each independently of the others H, OH, SH, NH₂, NHNH₂, NHOH, NHO-C₁-C₁₂alkyl, -N=CH-N(C₁-C₁₂alkyl)₂, -N=CH-N-cycloalkyl, F, Cl, Br, C₁-C₁₂alkyl, hydroxy-C₁-C₁₂alkyl, amino-C₁-C₁₂alkyl, C₁-C₁₂alkoxy, benzyloxy or C₁-C₁₂alkylthio, the hydroxy and amino groups being unsubstituted or substituted by a protecting group. phenyl, benzyl, primary amino having from 1 to 20 carbon atoms or secondary amino having from 2 to 30 carbon atoms, R_{b4} is hydrogen, CN or -C≡C-R_{b7}, and R_{b6} and R_{b7} are hydrogen or C₁-C₄alkyl.

44. An oligonucleotide according to claim 32, wherein B is a uracil, thymine or cytosine radical of formula III. IIIa. IIIb or IIIc 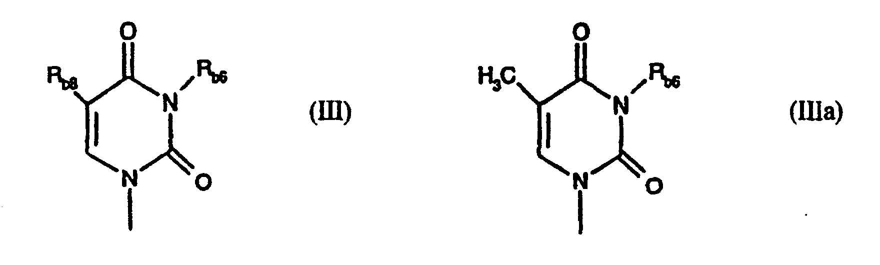 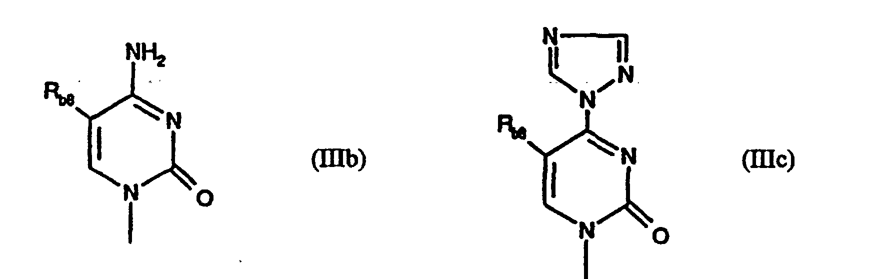 wherein R_{b6} is H or C₁-C₄alkyl and R_{b8} is H, OH, SH, NH₂, NHNH₂, NHOH, NHO-C₁-C₁₂alkyl, -N=CH-N(C₁-C₁₂alkyl)₂, -N=CH-N-cycloalkyl, F, Cl, Br, C₁-C₁₂alkyl, hydroxy-C₁-C₁₂alkyl, amino-C₁-C₁₂alkyl, C₁-C₁₂alkoxy, benzyloxy or C₁-C₁₂alkylthio, the hydroxy and amino groups being unsubstituted or substituted by a protecting group, phenyl, benzyl, primary amino having from 1 to 20 carbon atoms, secondary amino having from 2 to 30 carbon atoms, C₁-C₁₂alkenyl or C₁-C₁₂alkynyl, and the NH₂ group in formula IIIb is unsubstituted or is substituted by C₁-C₆alkyl, benzoyl or by a protecting group, or a dihydro derivative of a radical of formula III, IIIa, IIIb or IIIc.

45. An oligonucleotide according to claim 32, wherein R₃ is OH.

46. An oligonucleotide according to claim 32, wherein R₃ is F.

47. An oligonucleotide according to claim 32, wherein R₃ is (CF₂)ₙCF₃.

48. An oligonucleotide according to claim 32, wherein n is a number from 0 to 3.

49. An oligonucleotide according to claim 48, wherein n is 0.

50. Use of an oligonucleotide of formula V as a diagnostic agent for the *in vitro* detection of viral infections or genetic diseases.

51. A pharmaceutical composition comprising an effective amount of a nucleoside of formula I or of an oligonucleotide of formula V, on its own or together with other active ingredients, a pharmaceutical carrier and, where appropriate, excipients.

## Revendications

1. Composés de formule I : 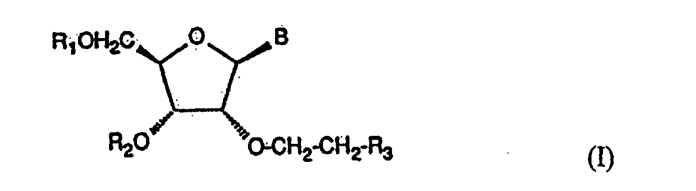 dans laquelle R₁ et R₂ représentent chacun indépendamment de l'autre un atome d'hydrogène ou un groupe protecteur, ou encore R₁ a ces significations et R₂ représente un radical phosphoré formant des groupes pontants nucléotidiques ; B est un résidu de purine ou de pyrimidine ou un analogue de ces derniers ; et R₃ est OH, F ou (CF₂)ₙCF₃, n étant un nombre de 0 à 7.

2. Composés selon la revendication 1, **caractérisés en ce que** R₁ et R₂ sont tous les deux des atomes d'hydrogène.

3. Composés selon la revendication 1, **caractérisés en ce que** R₁ et R₂ représentent des groupes protecteurs identiques.

4. Composés selon la revendication 1, dans lesquels B, en tant que résidu de purine ou analogue de ce dernier, représente un radical de formule II, IIa, IIb, IIc, IId, IIe ou IIf, 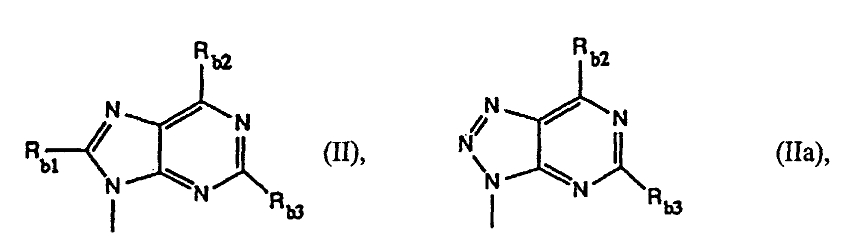 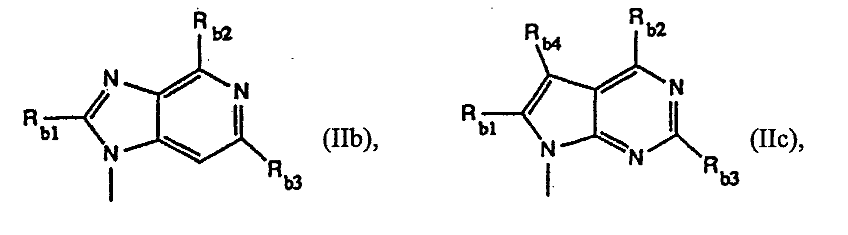 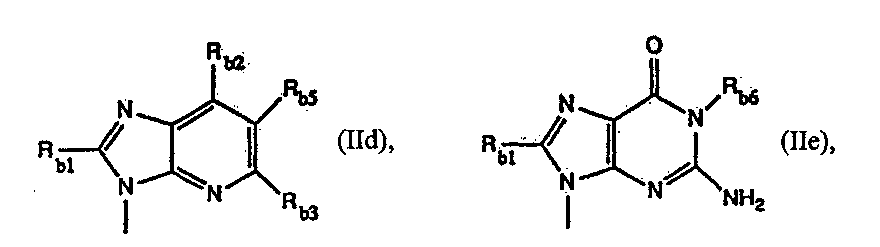 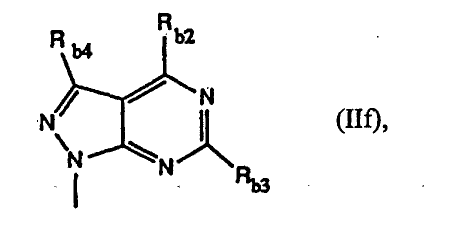 où R_{b1} est H, Cl, Br, OH ou un groupe -O-(alkyle en C₁-C₁₂), et R_{b2}, R_{b3} et Rb₅ représentent chacun indépendamment des autres H, OH, SH, NH₂, NHNH₂, NHOH, un groupe NHO-(alkyle en C₁-C₁₂), -N=CH-N(alkyle en C₁-C₁₂)₂, -N=CH-azacycloalkyle, F, Cl, Br, alkyle en C₁-C₁₂, hydroxyalkyle en C₁-C₁₂, aminoalkyle en C₁-C₁₂, alcoxy en C₁-C₁₂, benzyloxy, alkylthio en C₁-C₁₂, les groupes hydroxyle et amino étant non substitués ou étant substitués par un groupe protecteur, phényle, benzyle, amino primaire ayant de 1 à 20 atomes de carbone ou amino secondaire ayant de 2 à 30 atomes de carbone, R_{b4} est un atome d'hydrogène, CN ou -C≡C-R_{b7}, et R_{b6} et R_{b7} sont des atomes d'hydrogène ou des groupes alkyle en C₁-C₄.

5. Composés selon la revendication 4, dans lesquels le groupe protecteur des groupes hydroxyle et amino est un groupe acyle en C₁-C₈.

6. Composés selon la revendication 4, dans lesquels le groupe amino primaire contient de 1 à 12 atomes de carbone, et le groupe amino secondaire contient 2 à 12 atomes de carbone.

7. Composés selon la revendication 4, dans lesquels; pour ce qui concerne les groupes amino primaire et amino secondaire, il s'agit de radicaux de formule Rₐ₁Rₐ₂N, dans laquelle Rₐ₁ est H ou a d'une manière indépendante la signification de Rₐ₂, et Rₐ₂ est un groupe alkyle, aminoalkyle, hydroxyalkyle en C₁-C₂₀ ; carboxyalkyle ou carbalcoxyalkyle, où le groupe carbalcoxy contient de 2 à 8 atomes de carbone et le groupe alkyle contient de 1 à 6 et de préférence de 1 à 4 atomes de carbone ; alcényle en C₂-C₂₀ ; phényle, mono- ou di-(alkyle ou alcoxy en C₁-C₄)phényle, benzyle, mono- ou di-(alkyle ou alcoxy en C₁-C₄)benzyle ; ou encore 1,2-, 1,3- ou 1,4-imidazolyl-(alkyle en C₁-C₆), ou Rₐ₁ et Rₐ₂ forment ensemble un radical tétra- ou pentaméthylène, 3-oxa-1,5-pentylène,
-CH₂-NRₐ₃-CH₂CH₂- ou -CH₂CH₂-NRₐ₃-CH₂CH₂-, où Rₐ₃ est H ou un groupe alkyle en C₁-C₄, le groupe amino du groupe aminoalkyle étant non substitué ou étant substitué par un ou deux groupes alkyle ou hydroxyalkyle en C₁-C₄, et le groupe hydroxyle du groupe hydroxyalkyle étant éventuellement éthérifié par un groupe alkyle en C₁-C₄.

8. Composés selon la revendication 6, dans lesquels, pour ce qui concerne le groupe amino primaire et le groupe amino secondaire, il s'agit de groupes méthyl-, éthyl-, diméthyl-, diéthyl-, allyl-, mono- ou di-(hydroxy-éth-2-yl)-, phényl- et benzyl-, acétyl-, isobutyryl- et benzoylamino.

9. Composés selon la revendication 4, dans lesquels R_{b1}, dans les formules II, IIb, IIc, IId et IIe, représentent un atome d'hydrogène.

10. Composés selon la revendication 4, dans lesquels R_{b5}, dans la formule IId, est un atome d'hydrogène.

11. Composés selon la revendication 4, dans lesquels R_{b2} et R_{b3}, dans les formules II, IIa, IIb, IIc, IId et IIf, représentent chacun indépendamment de l'autre H, F, Cl, Br, OH, SH, NH₂, NHOH, NHNH_{z}, un groupe méthylamino, diméthylamino, benzoylamino, méthoxy, éthoxy et méthylthio.

12. Composés selon la revendication 4, dans lesquels B est un résidu de purine ou un résidu d'un analogue de la purine de la série comprenant l'adénine, la N-méthyladénine, la N-benzoyladénine, la 2-méthylthioadénine, la 2-aminoadénine, la 6-hydroxypurine, la 2-amino-6-chloropurine, la 2-amino-6-méthylthiopurine, la guanine et la N-isobutyrylguanine.

13. Composés selon la revendication 1, dans lesquels B, dans la formule I, et en tant que résidu de pyrimidine, représentent un radical uracile, thymine ou cytosine de formule III, IIIa, IIIb ou IIIc 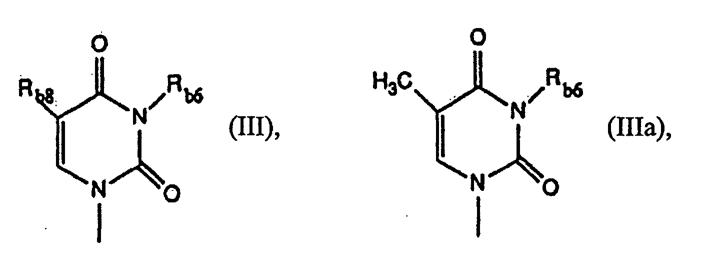 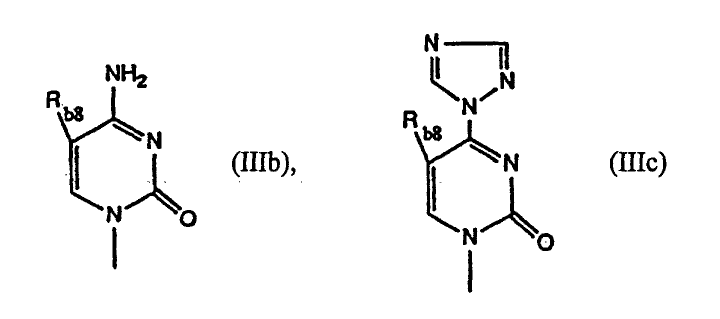 où R_{b6} est H ou un groupe alkyle en C₁-C₄, et R_{b8} est H, OH, SH, NH₂, NHNH₂, NHOH, un groupe NHO-(alkyle en C₁-C₁₂), -N=CH-N(alkyle en C₁-C₁₂)₂, -N=CH-azacycloalkyle, F, Cl, Br, alkyle en C₁-C₁₂, hydroxyalkyle en C₁-C₁₂, aminoalkyle en C₁-C₁₂, alcoxy en C₁-C₁₂, benzyloxy, alkylthio en C₁-C₁₂, les groupes hydroxyle et amino étant non substitués ou étant substitués par un groupe protecteur, phényle, benzyle, amino primaire ayant de 1 à 20 atomes de carbone, amino secondaire ayant de 2 à 30 atomes de carbone, alcényle en C₁-C₁₂ ou alcynyle en C₁-C₁₂, et le groupe NH₂ de la formule IIIb est non substitué ou est substitué par un groupe alkyle en C₁-C₆, benzoyle ou un groupe protecteur, et les dérivés dihydro des radicaux de formules III, IIIa, IIIb et IIIc.

14. Composés selon la revendication 13, dans lesquels R_{b8} est H ou un groupe alkyle ou hydroxyalkyle en C₁-C₆, alcényle ou alcynyle en C₂-C₆, F, Cl, Br, NH₂, benzoylamino, mono- ou di(alkyle en C₁-C₆)amino.

15. Composés selon la revendication 13, dans lesquels R_{b8} est H ou un groupe alkyle ou alcoxy ou hydroxyalkyle en C₁-C₆, alcényle ou alcynyle en C₂-C₆, F, Cl, Br, NH₂, benzoylamino, mono- ou di(alkyle en C₁-C₆)amino.

16. Composés selon la revendication 14, dans lesquels R_{b8} est H, F, Cl, Br, NH₂, NHCH₃, N(CH₃)₂, un groupe alkyle en C₁-C₄, alcényl-(1) en C₂-C₄ ou alcynyl-(1) en C₂-C₄.

17. Composés selon la revendication 15, dans lesquels R_{b8} est H ou un groupe alkyle en C₁-C₄, alcényl-(1) en C₂-C₄, alcynyl-(1) en C₂-C₄, NH₂, NHCH₃ ou (CH₃)₂N.

18. Composés selon la revendication 1, dans lesquels B, en tant que résidu d'un analogue de la pyridine, dérive de l'uracile, de la thymine, de la cytosine, du 5-fluorouracile, du 5-chlorouracile, du 5-bromouracile, du dihydrouracile, de la 5-méthylcytosine, de la 5-propynethymine et de la 5-propynecytosine.

19. Composés selon la revendication 1, **caractérisés en ce que** R₂, en tant que radical phosphoré formant un groupe pontant nucléotidique, correspond à la formule P1 ou P2 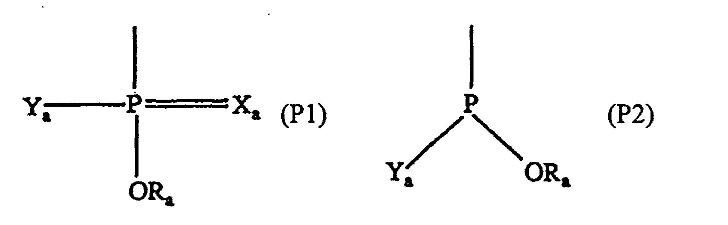 où Yₐ est un atome d'hydrogène, un groupe alkyle en C₁-C₁₂, aryle en C6-C₁₂, aralkyle en C₇-C₂₀, alcaryle en C₇-C₂₀, -OR_{b}, -SR_{b}, -NH₂, amino primaire, amino secondaire, O^{⊖}M^{⊕} ou S^{⊖}M^{⊕} ; Xₐ est un atome d'oxygène ou de soufre ; Rₐ est un atome d'hydrogène, M^{⊕}, un groupe alkyle en C₁-C₁₂, alcényle en C₂-C₁₂, aryle en C₆-C₁₂, ou encore le groupe RₐO-représente un groupe N-hétéroaryl-N-yle à 5 chaînons sur le noyau et 1 à 3 atomes d'azote ; R_{b} est un atome d'hydrogène, un groupe alkyle en C₁-C₁₂ ou aryle en C₆-C₁₂ ; et M^{⊕} est Na^{⊕}, K^{⊕}, Li^{⊕}, NH₄^{⊕} ou représente un ammonium primaire, secondaire, tertiaire ou quaternaire ; les groupes alkyle, aryle, aralkyle et alcaryle de Yₐ, Rₐ et R_{b} étant non substitués ou substitués par des substituants alcoxy, alkylthio, halogéno, -CN, -NO₂, phényle, nitrophényle ou halogénophényle.

20. Composés selon la revendication 19, **caractérisés en ce que** Rₐ est le radical β-cyanoéthyle et Yₐ le radical di(isopropyl)amino.

21. Composés selon la revendication 1, **caractérisés en ce que** R₃ est OH.

22. Composés selon la revendication 1, **caractérisés en ce que** R₃ est F.

23. Composés selon la revendication 1, **caractérisés en ce que** R₃ est (CF₂)ₙCF₃, et n est un nombre de 0 à 7.

24. Composés selon la revendication 23, **caractérisés en ce que** n est le nombre 0.

25. Procédé de préparation de composés de formule I 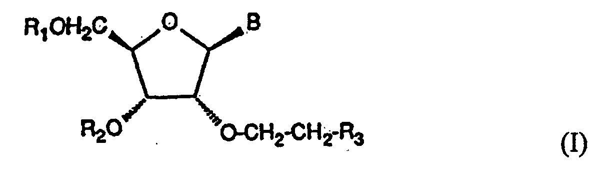 dans laquelle R₁ et R₂ représentent chacun indépendamment de l'autre un atome d'hydrogène ou un groupe protecteur ; B est un résidu de purine ou de pyrimidine ou un analogue de ces derniers ; et
(a) R₃ est OH,
**caractérisé en ce qu'**on fait réagir un composé de formule IVa 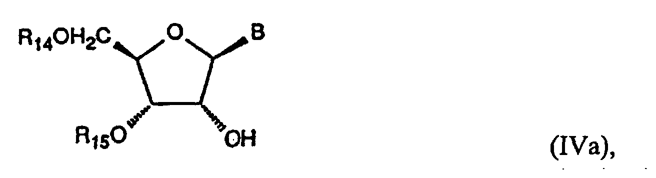 dans laquelle R₁₄ et R₁₅ représentent des groupes protecteurs identiques ou différents et B est un résidu de purine ou de pyrimidine ou un analogue de ce dernier, des groupes fonctionnels du résidu basique b étant protégés par des groupes protecteurs, dans un solvant inerte, avec un composé de formule A
X-CH₂-COOR₄ (A),
dans laquelle R₄ est un groupe alkyle en C₁-C₄ et X est Cl, Br, I, ou un groupe tosyl-O ou mésyl-O;
puis que l'on réduit la fonction ester avec du NaBH₄ ou du LiAlH₄, le groupe OH qui se forme pouvant être protégé d'une manière provisoire par un groupe tel que défini pour R₁ ;
(b) R₃ est F,
**caractérisé en ce qu'**on fait réagir un composé de formule I dans laquelle R₃ est OH avec un composé de formule B 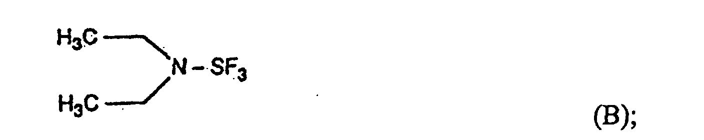
(c) R₃ est -(CF₂)ₙ-CF₃, où n est un nombre de 0 à 7,
**caractérisé en ce qu'**on fait réagir un composé de formule IVa avec un composé de formule C, D ou E
CH≡C-(CF₂)ₙ-CF₃ (C)
CH₂=CH-(CF₂)ₙ-CF₃ (D) 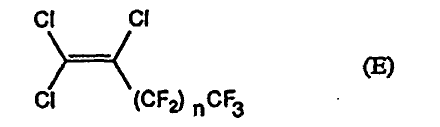
puis que l'on soumet à une réduction catalytique, pour obtenir du CH₂CH₂(CF)ₙCF₃, la double liaison ou la double liaison chlorée éventuellement présente ;
(d) R₃ est OH, F ou -(CF₂)ₙ-CF₃, où n est un nombre de 0 à 7,
**caractérisé en ce qu'**on substitue un composé de formule IVb 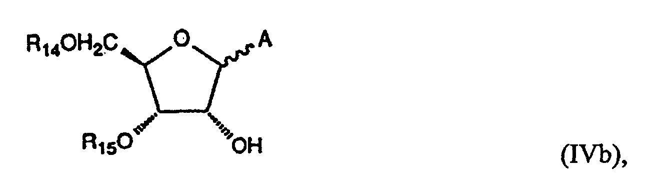 dans laquelle R₁₄ et R₁₅ ont les significations données ci-dessus et A est un groupe éliminable, sur le groupe 2'-OH par l'un des procédés décrits en (a) à (c), et que l'on introduit le résidu basique B par substitution ; et éventuellement on élimine les groupes protecteurs R₁₄ et R₁₅.

26. Utilisation des composés selon la revendication 1 pour préparer des oligonucléotides, qui contiennent des motifs monomères identiques ou différents de composés ayant les formules I ou qui contiennent au moins un motif monomère de composés de formules I en combinaison avec des motifs monomères d'autres nucléosides naturels ou synthétiques, les oligonucléotides comportant de 2 à 200 motifs monomères.

27. Utilisation selon la revendication 26 pour préparer des oligonucléotides ayant de 2 à 100 motifs monomères.

28. Utilisation selon la revendication 27, pour préparer des oligonucléotides ayant de 2 à 50 motifs monomères.

29. Utilisation selon la revendication 28, pour préparer des oligonucléotides ayant de 4 à 30 motifs monomères.

30. Utilisation selon la revendication 26, pour préparer des oligonucléotides ayant des motifs monomères, identiques ou différents, de composés de formules I.

31. Utilisation selon la revendication 26, pour préparer des oligonucléotides ayant des motifs monomères identiques de composés ayant les formules I et de motifs monomères de nucléosides naturels et synthétiques.

32. Oligonucléotides de formule V
5-U-(O-Y-O-V-)ₓO-Y-O-W-3' (V),
dans laquelle x est un nombre de 0 à 200 et Y est un groupe pontant nucléotidique ; U, V et W représentent des radicaux identiques ou différents de nucléosides naturels ou synthétiques, et au moins l'un des résidus U, V et/ou W représente un radical de formule VI 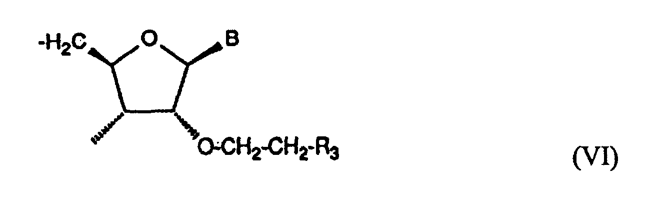 dans laquelle B est un résidu de purine ou de pyrimidine ou un analogue de ce dernier ; et R₃ est OH, F ou (CF₂)ₙCF₃, n étant un nombre de 0 à 7.

33. Oligonucléotides selon la revendication 32, **caractérisés en ce que** n est le nombre 0.

34. Oligonucléotides selon la revendication 32, **caractérisés en ce que**, pour ce qui concerne le groupe pontant Y, il s'agit de groupe -P(O)O^{⊖}-, -P(O)S^{⊖}-, -P(S)S^{⊖}-, -P(O)R₁₆-, -P(O)NR₁₇R₁₈- ou -CH₂-, où R₁₆ est H ou un groupe alkyle en C₁-C₆, et R₁₇ et R₁₈ ont chacun indépendamment de l'autre les significations de R₁₆.

35. Oligonucléotides selon la revendication 32, **caractérisés en ce que**, pour ce qui concerne le groupe pontant Y, il s'agit de -P(O)O^{⊖}-.

36. Oligonucléotides selon la revendication 32, **caractérisés en ce que** x est un nombre de 0 à 100.

37. Oligonucléotides selon la revendication 36, **caractérisés en ce que** x est un nombre de 1 à 50.

38. Oligonucléotides selon la revendication 37, **caractérisés en ce que** x est un nombre de 3 à 29.

39. Oligonucléotides selon la revendication 32, **caractérisés en ce que** les résidus de formule VI sont liés en position terminale et/ou dans la séquence nucléotidique.

40. Oligonucléotides selon la revendication 32, **caractérisés en ce que** les résidus de formule VI sont liés entre les radicaux de nucléosides naturels ou synthétiques.

41. Oligonucléotides selon les revendications 39 et 40, **caractérisés en ce que** 2 à 5 résidus identiques ou différents de formule VI se suivent l'un l'autre.

42. Oligonucléotides selon la revendication 32, **caractérisés en ce qu'**ils contiennent en tout 4 à 30 motifs nucléosidiques et 1 à 12 résidus de formule VI.

43. Oligonucléotides selon la revendication 32, dans lesquels B, en tant que résidu de purine ou analogues de ce dernier, représente un radical ayant les formules II, IIa, IIb, IIc, IId, IIe ou IIf 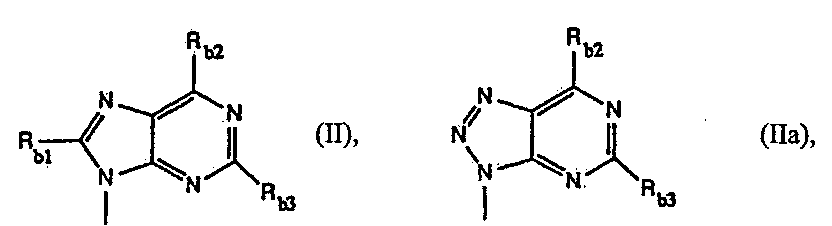 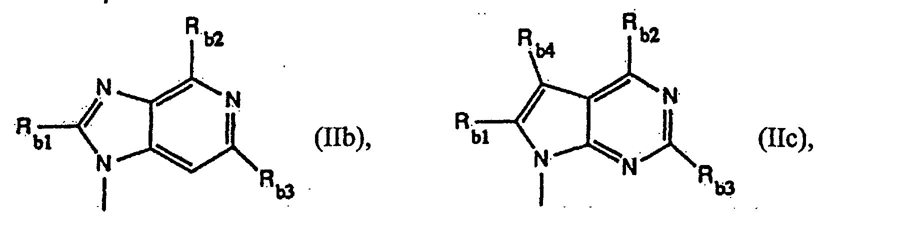 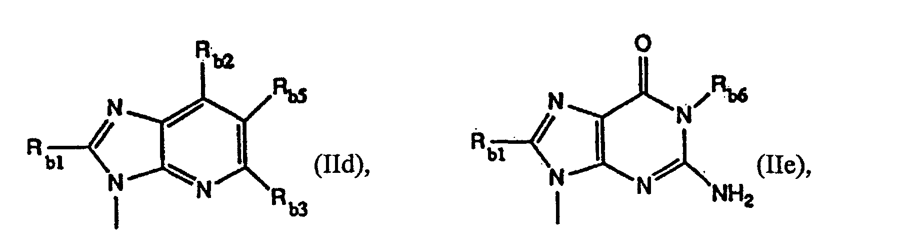 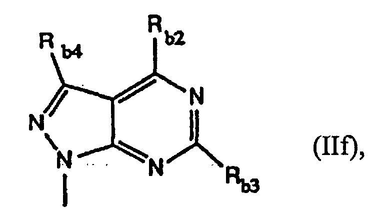 où R_{b1} est H, Cl, Br, OH ou un groupe -O-(alkyle en C₁-C₁₂), et R_{b2}, R_{b3} et Rb₅ représentent chacun indépendamment des autres H, OH, SH, NH₂, NHNH₂, NHOH, un groupe NHO-(alkyle en C₁-C₁₂), -N=CH-N(alkyle en C₁-C₁₂)₂, -N=CH-azacycloalkyle, F, Cl, Br, alkyle en C₁-C₁₂, hydroxyalkyle en C₁-C₁₂, aminoalkyle en C₁-C₁₂, alcoxy en C₁-C₁₂, benzyloxy, alkylthio en C₁-C₁₂, les groupes hydroxyle et amino étant non substitués ou étant substitués par un groupe protecteur, phényle, benzyle, amino primaire ayant de 1 à 20 atomes de carbone ou amino secondaire ayant de 2 à 30 atomes de carbone, R_{b4} est un atome d'hydrogène, CN ou -C≡C-R_{b7}, R_{b6} et R_{b7} sont des atomes d'hydrogène ou des groupes alkyle en C₁-C₄.

44. Oligonucléotides selon la revendication 32, dans lesquels B est un résidu d'uracile, de thymine ou de cytosine de formule III, IIIa, IIIb ou IIIc 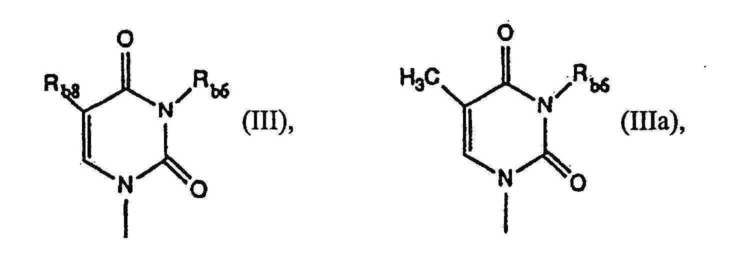 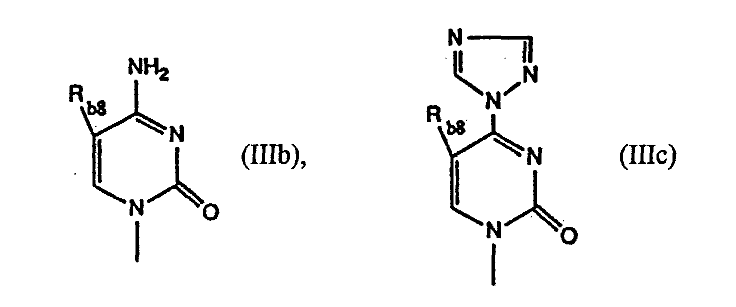 où R_{b6} est H ou un groupe alkyle en C₁-C₄, et R_{b8} est H, OH, SH, NH₂, NHNH₂, NHOH, un groupe NHO-(alkyle en C₁-C₁₂), -N=CH-N(alkyle en C₁-C₁₂)₂, -N=CH-cycloalkyle, F, Cl, Br, alkyle en C₁-C₁₂, hydroxyalkyle en C₁-C₁₂, aminoalkyle en C₁-C₁₂, alcoxy en C₁-C₁₂, benzyloxy, alkylthio en C₁-C₁₂, les groupes hydroxyle et amino étant non substitués ou étant substitués par un groupe protecteur, phényle, benzyle, amino primaire ayant de 1 à 20 atomes de carbone, amino secondaire ayant de 2 à 30 atomes de carbone, alcényle en C₁-C₁₂ ou alcynyle en C₁-C₁₂, et le groupe NH₂ de la formule IIIb est non substitué ou est substitué par un groupe alkyle en C₁-C₆, benzoyle ou un groupe protecteur, et les dérivés dihydro des radicaux de formules III, IIIa, IIIb et IIIc.

45. Oligonucléotides selon la revendication 32, dans lesquels R₃ est OH.

46. Oligonucléotides selon la revendication 32, dans lesquels R₃ est F.

47. Oligonucléotides selon la revendication 32, dans lesquels R₃ est (CF₂)ₙCF₃.

48. Oligonucléotides selon la revendication 32, dans lesquels n est un nombre de 0 à 3.

49. Oligonucléotides selon la revendication 48, dans lesquels n vaut 0.

50. Utilisation des oligonucléotides de formule V en tant que produits diagnostiques pour la détection in vitro d'infections virales ou de maladies d'origine génétique.

51. Préparation pharmaceutique contenant une quantité efficace d'un nucléoside ayant les formules I ou d'un oligonucléotide de formule V, seul ou en même temps que d'autres principes actifs, un matériau support pharmaceutique et éventuellement des adjuvants.
